# EUROPÄISCHE PATENTSCHRIFT

(11) **EP 2 519 238 B1**
(45) Veröffentlichungstag und Bekanntmachung des Hinweises auf die Patenterteilung: **13.08.2014**
(21) Anmeldenummer: 10812791.1
(22) Anmeldetag: 28.12.2010
(51) Int. Cl.: A61K 31/437, A61K 9/00, A61K 9/20, A61P 27/16, A61P 1/08, A61P 25/00

(54) **BETA-CARBOLINE ZUR BEHANDLUNG VON HÖRSCHÄDEN UND SCHWINDEL**
BETA-CARBOLINES FOR THE TREATMENT OF HEARING LOSS AND VERTIGO
BETA-CARBOLINE POUR TRAITER DES TROUBLES DE L'AUDITION ET LES VERTIGES

(30) Priorität: 02.02.2010 US 282395 P; 28.12.2009 DE 102009060811
(43) Veröffentlichungstag der Anmeldung: 07.11.2012
(73) Patentinhaber: Audiocure Pharma GmbH, 14193 Berlin (DE)
(72) Erfinder: Rommelspacher, Hans, 14193 Berlin (DE)
(74) Vertreter: Arth, Hans-Lothar
(86) Internationale Anmeldenummer: PCT/DE2010/001530
(87) Internationale Veröffentlichungsnummer: WO 2011/079841

(56) Entgegenhaltungen:
- DE-A1-102007 009 264
- US-A1- 2004 038 970

## Beschreibung

Die vorliegende Erfindung betrifft β-Carboline der allgemeinen Formel (I) bevorzugt 9-Alkyl-β-Carboline (9-Alkyl-BC) und pharmazeutische Zusammensetzungen enthaltend diese Verbindungen zur Prophylaxe und Behandlung von Hörschäden, Schwindel und Gleichgewichtsstörungen. Die Erfindung wird durch die beigefügten Ansprüche definiert.

### Hintergrund der Erfindung

Die erfindungsgemäßen Verbindungen dienen zur Behandlung von akuten und - chronischen Ohrenerkrankungen und Hörschäden, Schwindel und Gleichgewichtsstörungen insbesondere von Hörsturz, Knalltrauma, Explosiontrauma, Innenohrschwerhörigkeit durch chronische Lärmexposition, Altersschwerhörigkeit, Trauma während der Implantation von Innenohrprothesen (Insertionstrauma), Schwindel aufgrund von Erkrankungen im Innenohrbereich, Schwindel im Zusammenhang und/oder als Symptom der Meniere'schen Erkrankung, Gleichgewichtsstörungen im Zusammenhang und/oder als Symptom der Meniere'schen Erkrankung, Tinnitus und Hörschäden aufgrund von Antibiotika und Zytostatika.

Nach Erhebungen der Weltgesundheitsorganisation (WHO) leiden ca. 250 Millionen Menschen weltweit an leichten oder schweren Hörstörungen. In den USA sind 30 bis 40 Millionen Menschen von Hörschädigungen und von Hörverlusten betroffen. Allein hier betragen die Kosten der Behandlungen ca. 50 Milliarden USD jährlich. Der deutsche Schwerhörigenbund berichtete im Jahr 2007, dass ca. 19% der deutschen Bevölkerung über 14 Jahre an Hörstörungen leiden.

Mit zunehmendem Alter nimmt der Prozentsatz an Hörgeschädigten zu. Bereits jetzt stellen die Hörstörungen bei über 65-jährigen Menschen die vierthäufigste chronische körperliche Beeinträchtigung, nach Erkrankungen der Knochen und Gelenke, Bluthochdruck und Herzkrankheiten, dar. Unter den 60 bis 69-jährigen sind 37%, unter den 70-jährigen und älteren über 54% der Bevölkerung von Hörschädigungen betroffen.

In der Bundesrepublik Deutschland leiden ca. 12-15 Millionen Patienten an Innenohrschwerhörigkeit und ca. 2,9 Millionen Patienten an Tinnitus.

Der Begriff "Tinnitus aurium" (lat. "das Klingeln der Ohren") oder kurz Tinnitus bezeichnet ein Symptom oder auch Syndrom, bei dem der Betroffene Geräusche wahrnimmt, die keine äußere für andere Personen wahrnehmbare Quelle besitzen. Im Gegensatz dazu beruht der "objektive Tinnitus" auf einer von außen wahrnehmbaren oder zumindest messbaren körpereigenen Schallquelle. Objektiver Tinnitus ist allerdings im Vergleich zum subjektiven Tinnitus sehr selten.

Der Tinnitus ist eine akustische Wahrnehmung, die unabhängig von einem Schall, der auf das Ohr wirkt, vom Patienten wahrgenommen wird. Diese Wahrnehmung beruht auf einer Störung der Hörfunktion des Innenohrs. Der Höreindruck des Tinnitus hat also nichts mit dem Schall in der Umgebung des Patienten zu tun. Die Art der scheinbaren Geräusche, welche der Patient wahrnimmt, ist sehr vielfältig. Man fasst unter anderen folgende akustische Eindrücke unter dem Begriff Tinnitus zusammen:
- Brumm- oder Pfeiftöne
- Zischen
- Rauschen
- Knacken oder Klopfen

Das Geräusch kann in seiner Intensität gleichbleibend sein, es kann jedoch auch einen rhythmisch-pulsierenden Charakter haben. Es gibt nicht immer ein reales Geräusch, das denselben Höreindruck wie der Tinnitus verursacht. Auch sollte man Tinnitus deutlich von akustischen Halluzinationen abgrenzen.

Etwa 10-20 % der Bevölkerung sind von Tinnitus dauerhaft betroffen, knapp 40 % stellen zumindest einmal im Leben ein derartiges Ohrgeräusch fest. Etwa ein Drittel aller älteren Menschen gibt an, ständig Ohrgeräusche wahrzunehmen. Der Beginn der Krankheit liegt typischerweise zwischen dem 40. und 50. Lebensjahr, wobei Frauen und Männer gleichermaßen betroffen sind. Besonders in den letzten Jahrzehnten ist die Zahl der Tinnituspatienten in den westlichen Industrieländern stark gestiegen.

DE102007009264 offenbart 9-Alkyl-β-Carboline der allgemeinen Formel (I), sowie pharmazeutische Zusammensetzungen, die diese Formel enthalten, zur Behandlung und Prophylaxe von Bewegungsstörungen, neurodegenerative Erkrankungen, Alzheimer und Parkinsonschen Krankheit.

US20040038970 offenbart β-Carboline zur Behandlung von Tinnitus.

Der vorliegenden Erfindung liegt die Aufgabe zugrunde, Wirkstoffe sowie pharmazeutische Formulierungen bereitzustellen, welche zur Prophylaxe und Behandlung von Hörschäden, Altersschwerhörigkeit, Schwindel und Gleichgewichtsstörungen eingesetzt werden können.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen, der Beschreibung und den Beispielen.

### Beschreibung

Die Erfindung betrifft Verbindungen der allgemeinen Formel (I) 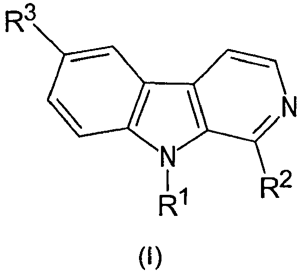 worin
R¹ für -R¹² steht;
R² und R³ unabhängig voneinander folgende Reste bedeuten: -R⁷, -R⁸, -H, -OH, -OR⁷, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃,, -F, -Cl, -Br, -I, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -OCF₃, -OC₂F₅.
**R⁷**, **R⁸** und **R¹²** unabhängig voneinander folgende Reste bedeuten: -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-C₆H₁₁, -cyclo-C₇H₁₃, -cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)2, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH₌CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅. -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂,, -CH₂-CH=CH-CH₂-CH=CH₂, -CH=CH-C₂H₄-CH=CH₂,, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂,, -CH=CH-C(CH₃)=CH-CH₃, -CH=C(CH₃)-CH=CH-CH₃, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C₁₄H₂₉, -CH₂-CH₂-N(CH₃)₂,
sowie pharmakologisch verträgliche Salze, Solvate, Hydrate, Enantiomere, Diastereomere, Mischungen von Diastereomeren, Tautomere als auch Racemate der vorgenannten Verbindungen zur Prophylaxe und Behandlung von Hörschäden, Schwindel und Gleichgewichtsstörungen.

Der hierin verwendete Ausdruck "Tautomer" ist als organische Substanz definiert, die durch eine chemische Reaktion, die Tautomerisierung, in ihr Gleichgewichtsisomer überführt werden kann. Die Tautomerisierung kann bevorzugt von Basen, Säuren oder anderen geeigneten Substanzen katalysiert werden.

### Allgemeine Synthese von 9-Alkyl-β-Carbolinen

Die Ausgangsverbindung Norharman kann nach literaturbekannten Vorschriften beispielsweise wie in Beispiel 1 beschrieben hergestellt werden.

Die N-Alkylierung in Position 9 erfolgt gemäß gängiger Alkylierungsreaktionen mittels Alkyliodiden, Alkylbromiden, Alkylchloriden, Alkylmesylaten, Alkyltosylaten oder anderen Alkylierungsreagenzien gemäß folgendem Reaktionsschema:

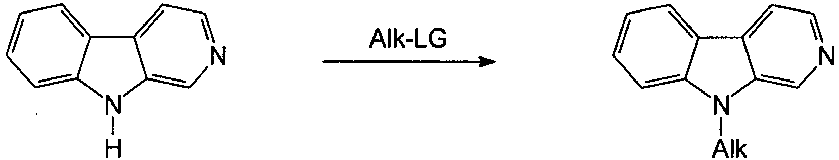

LG steht für eine Abgangsgruppe. Die Alkylierungsreaktionen sind vorzugsweise basenkatalysiert.

### Allgemeine Reaktionsvorschrift zur Alkylierung:

1 mol Equivalent Norharman wird in einem getrockneten Lösungsmittel wie z.B. DMF, THF, Methylenchlorid, usw. unter Schutzgasatmosphäre gelöst. Die Deprotonierung erfolgt mittels eines Überschusses einer starken Base, vorzugsweise Natriumhydrid (ca. 2 mol Equivalente) bei erniedrigter Temperatur (0°C bis -78°C). Ebenfalls bei einer Temperatur unter 0°C wird 1,0 bis 1,2 mol Equivalente eines Alkylierungsmittels zugesetzt, welches in einem getrockneten Lösungsmittel gelöst sein kann. Man rührt die Mischung über Nacht, wobei sich die Reaktionslösung auf Raumtemperatur erwärmen kann. Die Aufarbeitung erfolgt in einer dem Fachmann bekannten Weise. Nicht umgesetztes Norharman kann durch eine lonenaustausch-Chromatographie oder lonenpaar-Extraktion entfernt werden. Es ergeben sich in der Regel Ausbeuten von 30 bis 75% der Theorie.

Folgende Verbindungen wurden gemäß obiger Alkylierung hergestellt.

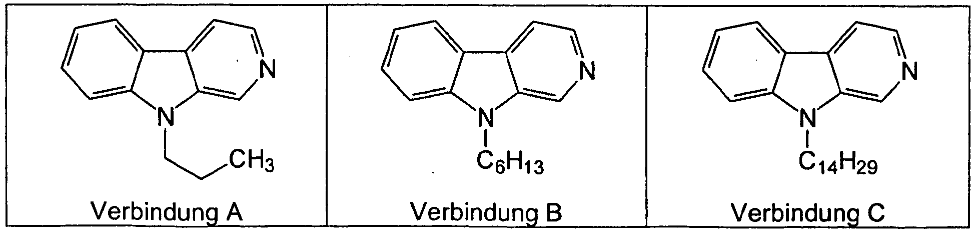

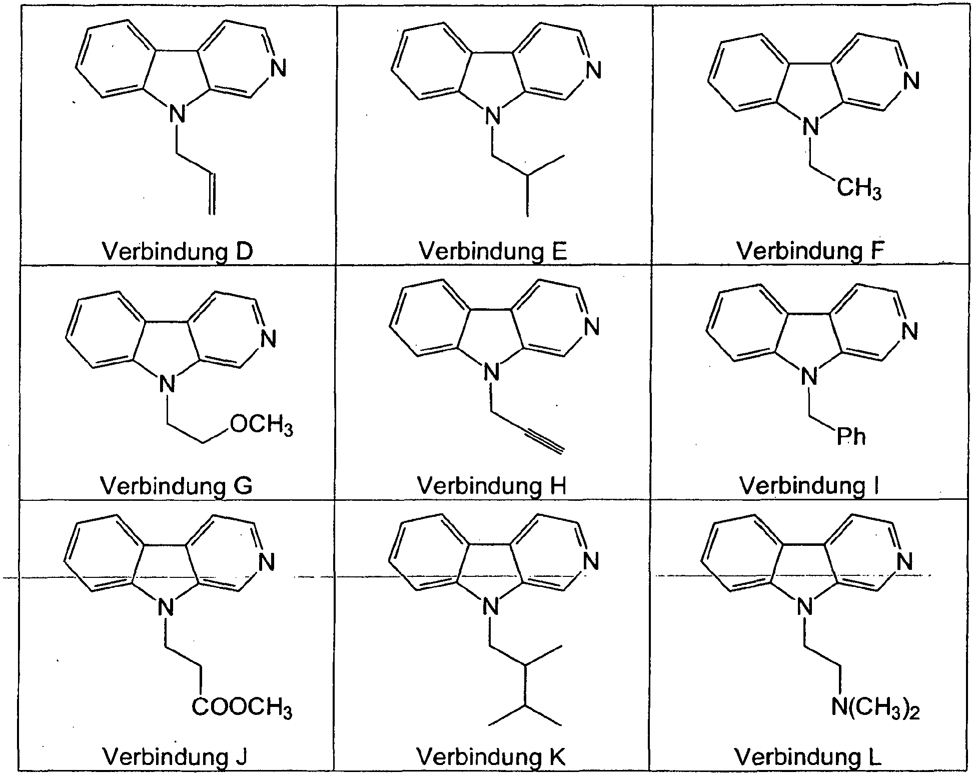

### Allgemeine Synthese von 1,6-disubstituierten β-Carbolinen

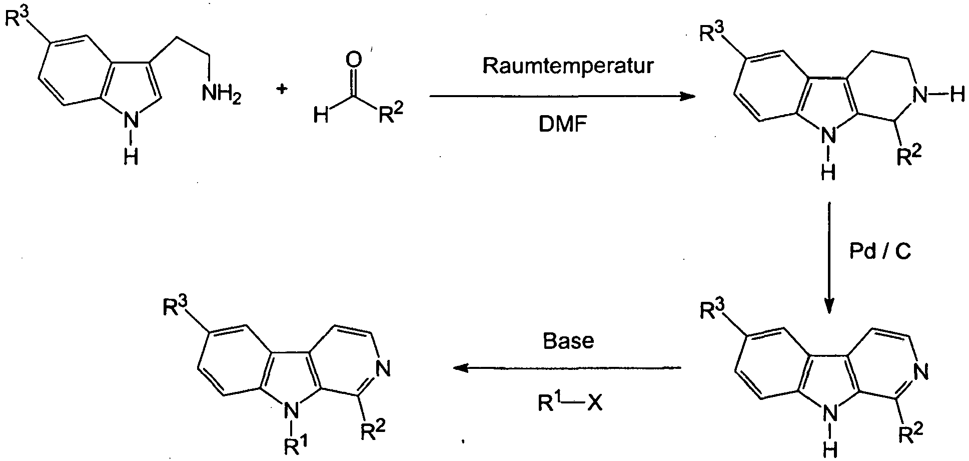

Um 1,6-disubstituierte β-Carboline zu erhalten, wurden Indolderivate mit den entsprechenden Aldehyden gemäß obigem Reaktionsschema umgesetzt.

Dabei wurden 0,1 Mol des Indolderivates in DMF gelöst und 0,12 Mol des Aldehyds unter Rühren hinzugefügt. Die Reaktionsmischung wurde 16 h bei Raumtemperatur gerührt. Nach Entfernen des Lösungsmittels wurde der erhaltene Feststoff zwei Mal in Toluol umkristallisiert und getrocknet. In einem weiteren Syntheseschritt wurden 0,07 Mol des aus Toluol umkristallisierten und getrockneten Feststoffes in 600 ml Cumol gelöst und unter Stickstoffatmosphäre mit 2,6 g Pd/C (10 %) für 90 min. Rückfluss erhitzt. Nach Zugabe von 100 ml Ethanol wurde die heiße Lösung filtriert und die Kohle mit 3 x 30 ml heißem Ethanol extrahiert. Die vereinigten flüssigen Fraktionen wurden unter Vakuum vom Lösungsmittel befreit und der Rückstand aus Toluol kristallisiert, um das 1,3-disubstituierte Norharman-Derivat zu erhalten.

Die entsprechenden 1-substituierten β-Carboline werden erhalten, wenn R³ ein Wasserstoffatom ist.

Die N-Alkylierung an Position 9 wird nach der Ringschlussreaktion mittels einer Base vorzugsweise eines Hydrids und anschließender Zugabe eines Alkylierungsmittels z.B. einer Alkyliodids vorgenommen. Eine ausführliche Vorschrift ist im experimentellen Teil enthalten. Gemäß dieser Reaktionsvorschrift wurden die folgenden Verbindungen hergestellt.

Bevorzugt sind folgende Verbindungen der allgemeinen Formeln (II) - (V) und (XXI)

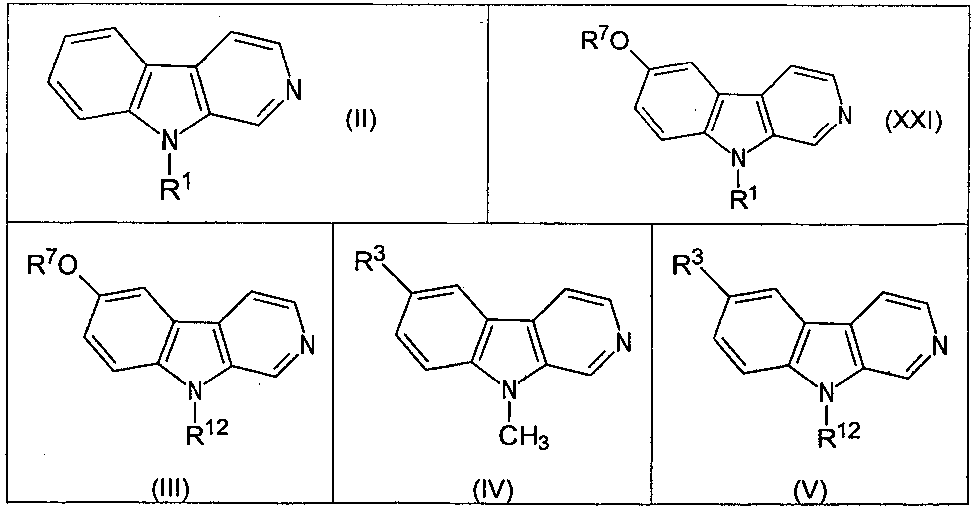

Die erfindungsgemäßen 9-Alkyl-β-Carboline zeigen überraschenderweise eine pharmakologische Wirkung bei Hörschäden, Schwindel und Gleichgewichtsstörungen sowie anderen Ohrenerkrankungen und werden somit erfindungsgemäß zur Prophylaxe und Behandlung von Hörschäden, Schwindel und Gleichgewichtsstörungen eingesetzt. Die Indikationen Hörschäden, Schwindel und Gleichgewichtsstörungen umfassen vor allem auch Innenohrschwerhörigkeit, Altersschwerhörigkeit, Hörsturz, Hörverlust, Trauma während der Implantation von Innenohrprothesen (Insertionstrauma), Innenohrschwerhörigkeit durch chronische Lärmexposition, Knalltrauma, Drehschwindelanfälle, Übelkeit und Erbrechen aufgrund von Hörschäden, Schwindel aufgrund von Erkrankungen im Innenohrbereich, Schwindel im Zusammenhang und/oder als Symptom der Menière'schen Erkrankung, Gleichgewichtsstörungen im Zusammenhang und/oder als Symptom der Menière'schen Erkrankung, Tinnitus und Hörschäden aufgrund von Antibiotika wie beispielsweise Penicilline wie z.B. Penicillin V, Propicillin, Azidozillin; Aminopenicilline wie z.B. Ampicillin, Amoxicillin; Cephalosporine wie z.B. Cefaclor, Cefradin; Lincomycine wie z.B. Lincomycin, Clindamycin; Tertracycline wie z.B. Doxycyclin, Tetrazyclin; Nitroimidazole wie z.B. Metronidazol; Makrolide wie z.B. Erythromycin; Aminoglykoside wie z.B. Gentamycin, Streptomycin und Zytostatika wie beispielsweise Actinomycin D, Aminoglutethimid, Amsacrin, Anastrozol, Antagonisten von Purin- und Pyrimidin-Basen, Anthracycline, Aromatasehemmer, Asparaginase, Antiöstrogene, Bexaroten, Bleomycin, Buselerin, Busulfan, Camptothecin-Derivate, Capecitabin, Carboplatin, Carmustin, Chlorambucil, Cisplatin, Cladribin, Cyclophosphamid, Cytarabin, Cytosinarabinosid, alkylierende Cytostatika, Dacarbazin, Dactinomycin, Daunorubicin, Docetaxel, Doxorubicin (Adriamycin), Epirubicin, Estramustin, Etoposid, Exemestan, Fludarabin, Fluorouracil, Folsäureantagonisten, Formestan, Gemcitabin, Glucocorticoide, Goselerin, Hormone und Hormonantagonisten, Hycamtin, Hydroxyharnstoff, Idarubicin, Ifosfamid, Imatinib, Irinotecan, Letrozol, Leuprorelin, Lomustin, Melphalan, Mercaptopurin, Methotrexat, Miltefosin, Mitomycine, Mitosehemmstoffe, Mitoxantron, Nimustine, Oxaliplatin, Paclitaxel, Pentostatin, Procarbazin, Tamoxifen, Temozolomid, Teniposid, Testolacton, Thiotepa, Tioguanin, Topoisomerase-Inhibitoren, Topotecan, Treosulfan, Tretinoin, Triptorelin, Trofosfamide, Vinblastin, Vincristin, Vindesin, Vinorelbin, zytostatisch wirksame Antibiotika.

Die Indikationen Hörschäden und Hörverlust können auch lärminduziert sein und/oder im Zusammenhang mit Knalltrauma und/oder Explosiontrauma. Von den am meisten genannten Faktoren sind das akute akkustische Trauma wie bei einem Knalltrauma oder Explosionstrauma, Lärmbelastung am Arbeitsplatz und in der Freizeit.

Tinnitus und Hörschäden können auch auf vaskulärer Ischämie, autoimmunologischen Erkrankungen, Infektionskrankeiten, Otosclerose und Schädeltrauma beruhen. Somit betrifft die vorliegende Erfindung auch Hörschäden und Hörverlust verursacht durch Knalltrauma, Explosiontrauma, Lärmbelastung, vaskuläre Ischämie, autoimmunologische Erkrankungen, Infektionskrankeiten, Otosclerose und Schädeltrauma.

Schwindel im Zusammenhang mit der Menière'schen Erkrankung ist eine erfindungsgemäß adressierbare Indikation, da bei dieser Form des Schwindels das Innenohr beteiligt ist. Dies gilt auch für Störungen des Gleichgewichts, da das Labyrinth als Gleichgewichtsorgan Teil des Innenohrs ist. Die Menière'sche Erkrankung oder Morbus Menière äußert sich in plötzlichen auftretenden Drehschwindelanfällen, Übelkeit, Erbrechen, Ohrensausen (Tinnitus aurium) und einseitigem Gehörverlust. Plötzlich auftretender Drehschwindel mit Übelkeit bis zum Erbrechen, die ohne erkennbaren Anlass zu jeder Tages- und Nachtzeit auftreten können, sind Kennzeichen dieser Erkrankung. Sie dauern minuten- bis stundenlang an und wiederholen sich in unterschiedlich großen Abständen. Das Schwindelgefühl kann so stark ausgeprägt sein, dass der Patient selbsttätig nicht mehr stehen kann. Zusätzlich besteht ein fluktuierender (zeitweise auftretender) Hörverlust verbunden mit Ohrensausen (Tinnitus) und einem Druckgefühl im betroffenen Ohr.

Bevorzugt sind Substituenten an den Positionen 9 (R¹) und 6 (R³) des β-Carbolin-Ringsystems. Das Substitutionsmuster in R¹ umfasst bevorzugt Alkylsubstituenten, besonders bevorzugt den Methylsubstituenten. Die Reste R² und R³ umfassen besonders bevorzugt Alkylsubstituenten, Halogene und Alkoxysubstituenten. R³ ist vorzugsweise ein Alkoxyrest wie beispielsweise -OCF₃, -OCH₂-CH₂F, -OCH₂-CF₃, -OCH₂-CH₂Cl, -O-cyclo-C₃H₅, -O-cyclo-C₅H₉, -O-cyclo-C₆H₁₁, -OPh, -OCH₂-Ph, -OCH₃, -OC₂H₅, -OC₃H₇, -OCH(CH₃)₂, -OC₄H₉, -OC₅H₁₁, -OCH(C₂H₅)₂, -OC₆H₁₃, -OCH=CH₂, -OCH₂-CH=CH₂, -OC₇H₁₅, -OC₈H₁₇, -OCH₂-CH=CH-CH₃, -OCH₂OH, -OCH₂-CH₂NH₂, -OCH₂-CH₂-CH₂OH, -OCH₂-CH₂-OCH₃, -OCH₂-CH₂OH, -OCH₂-OCH₃, -OCH₂-C≡CH.

R¹ ist vorzugsweise ein Alkylrest mit bis zu 6 Kohlenstoffatomen, weiter bevorzugt mit bis zu 4 Kohlenstoffatomen.

Insbesondere bevorzugt sind die Sibstanzen 9-Methyl-β-Carbolin und 9-Fluorethyl-ß-Carbolin.

Bevorzugte Verbindugen sind:

| | | |
|---|---|---|
| 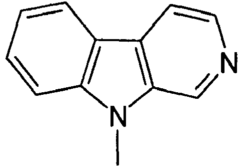 | 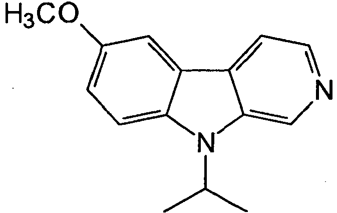 | 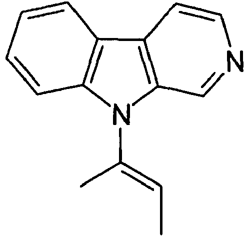 |
| 9-Methyl-9*H*-β-Carbolin (9-Me-BC) (VI) | 6-Methoxy-9-iso-Propyl-9*H*-β-Carbolin (VII) | 9-[(1*Z*)-1-Methylprop-1-enyl]-9*H*-β-Carbolin (VIII) |
| 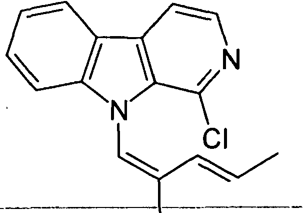 | 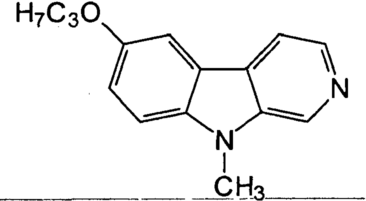 | 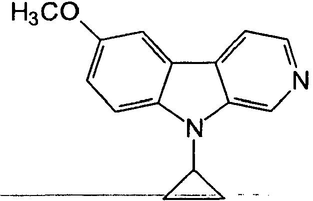 |
| 1-Chlor-9-[(1*Z*,3*E*)-2-Methylpenta-1,3-dienyl]-9*H*-β-Carbolin (IX) | 9-Methyl-6-Propoxy-9*H*-β-Carbolin (X) | 9-Cyclopropyl-6-Methoxy-9*H*-β-Carbolin (XI) |
| 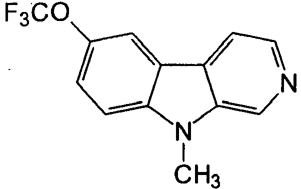 | 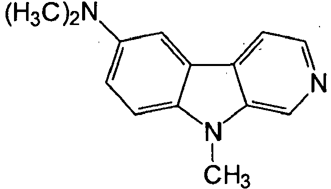 | 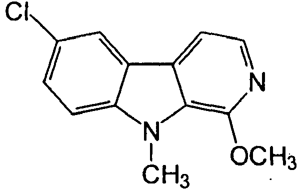 |
| 6-Trifluormethoxy-9-Methyl-9*H*-β-Carbolin (XII) | 6-Dimethylamino-9-Methyl-9*H*-β-Carbolin (XIII) | 1-Methoxy-6-Chlor-9-Methyl-9*H*-β-Carbolin (XIV) |
| 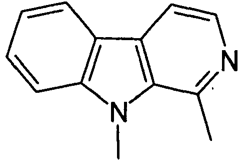 | 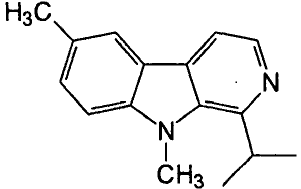 | 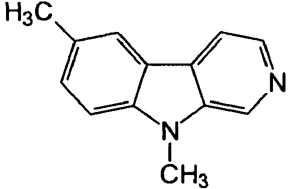 |
| 1,9-Dimethyl-9*H*-β-Carbolin (XV) | 1-Isopropyl-6,9-Dimethyl-9*H-*β-Carbolin (XVI) | 6,9-Dimethyl-9*H*-β-Carbolin (XVII) |
| 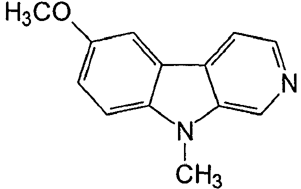 | 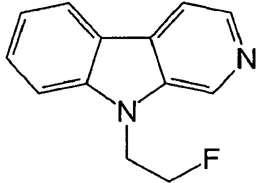 | 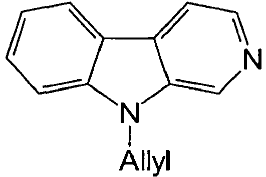 |
| 6-Methoxy-9-Methyl-9*H*-β-Carbolin (XVIII) | 9-(2-Fluorethyl)-9*H*-β-Carbolin (XIX) | 9-Allyl-9*H*-β-Carbolin (XX) |

Die Verbindungen der vorliegenden Erfindung und insbesondere die bevorzugten Verbindungen der allgemeinen Formeln (II) - (XX) sind geeignet zur Verwendung in der Behandlung und/oder Prophylaxe von akuten und chronischen Ohrenerkrankungen und Hörschäden, Schwindel und Gleichgewichtsstörungen insbesondere von Hörsturz, Knalltrauma, Innenohrschwerhörigkeit durch chronische Lärmexposition, Altersschwerhörigkeit, Trauma während der Implantation von Innenohrprothesen (Insertionstrauma), Schwindel aufgrund von Erkrankungen im Innenohrbereich, Schwindel im Zusammenhang und/oder als Symptom der Menière'schen Erkrankung, Gleichgewichtsstörungen im Zusammenhang und/oder als Symptom der Menière'schen Erkrankung, Tinnitus und Hörschäden aufgrund von Antibiotika und Zytostatika eingesetzt werden

Die Verbindungen entsprechend der vorliegenden Erfindung können selbst oder in Form eines pharmakologisch wirksamen Salzes verabreicht werden. Da die Verbindungen der vorliegenden Erfindung basische Eigenschaften besitzen können, können nach gängigen Methoden Salze dieser Verbindungen hergestellt werden.

Als Säuren, welche ein Säureadditionssalz mit den Verbindungen der vorliegenden Erfindung bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure.

Je nach Art der Verbindung sind auch Betain-Formen möglich.

Ferner betrifft die vorliegende Erfindung pharmazeutische Zusammensetzungen, welche unter Verwendung mindestens einer erfindungsgemäßen Verbindung oder eines Salzes davon hergestellt wurden.

Neben mindestens einer Verbindung der vorliegenden Erfindung enthalten die pharmazeutischen Zusammensetzungen einen pharmakologisch verträglichen Träger, Hilfsstoff und/oder Lösungsmittel.

Derartige Formulierungen sind unter anderem für die Inhalation oder die intravenöse, intraperitoneale, intramuskuläre, subkutane, mucokutane, orale, rektale, transdermale, topikale, bukkale, intradermale, intragastrale, intrakutane, intranasale, intrabuccale, perkutane oder sublinguale Verabreichung geeignet. Besonders bevorzugt ist die Verabreichung bzw. Injektion in das Mittelohr sowie die topische Verabreichung auf das Trommelfell.

Die pharmazeutischen Zusammensetzungen können in Form von transdermalen Applikationssystem (Pflaster, Film), Tropfen, Pillen, Tabletten, Filmtabletten, Schichttabletten, Gelen, Salben, Sirup, Granulaten, Suppositorien, Emulsionen, Dispersionen, Mikrokapseln, Kapseln, Puder oder Injektionslösungen hergestellt und verabreicht werden. Bevorzugt sind pharmazeutische Formulierungen in Form von Liposomen, Gelen und Emulsionen.

Das Ohr ist folgendermaßen aufgebaut. Von der Ohrmuschel aus führt der Gehörgang in das Innere des Ohrs. Der Gehörgang endet am Trommelfell. Diesen Teil des Ohrs bezeichnet man als Äußeres Ohr. Hinter dem Äußeren Ohr liegt ein Raum, das sogenannte Mittelohr, welches mit der Eustachischen Röhre verbunden ist. Das Mittelohr wird auf seiner einen Seite vom Trommelfell abgeschlossen und auf der gegenüber liegenden Seite durch das Ovale Fenster und durch das Runde Fenster. Hinter den beiden Fenstern schließt sich der Innenohr genannte Raum an. Das Innenohr beherbergt mehrere Organe des Ohrs, u. a. die sogenannte Schnecke (Cochlea). Diese wiederum enthält das Cortische Organ. Sowohl die Schnecke als auch das Cortische Organ grenzen direkt an das Runde Fenster. Die Schnecke ist außerdem verbunden mit dem Hörnerv.

Die Membran des Runden Fensters bildet also die biologische Barriere zum Innenohrraum und stellt das größte Hindernis für die lokale Therapie der Hörschädigung dar. Der verabreichte Arzneistoff muss diese Membran überwinden, um in den Innenohrraum zu gelangen. Der Arzneistoff kann aber nicht mechanischapparativ durch die Membran appliziert werden, weil sonst die Membran durch die Manipulation geschädigt wird. Er kann jedoch operativ (z.B. Injektion durch das Trommelfell) an der Rundfenstermembran lokal platziert werden und kann dann durch die Rundfenstermembran penetrieren. Die Sinneszellen (innere und äußere Haarzellen) des Hörorgans befinden sich in der Schnecke und werden in Ihrer Gesamtheit als Cortisches Organ bezeichnet. Die Haarzellen sind bei Innenohrschwerhörigkeit jeglicher Ursache (Alter, Medikamente wie beispielsweise bestimmte Antibiotika und Zytostatika) und bei Tinnitus primär geschädigt. Das runde Fenster schließt die Scalae tympani und vestibuli ab, bei denen es sich um Kompartimente innerhalb der Schnecke handelt. Die Scalae tympani und vestibuli sind mit der Perilymphe und der Endolymphe gefüllt, so dass die Haarzellen in direktem Kontakt mit der Perilymphe stehen. Daher verteilen sich Substanzen, die durch das runde Fenster dringen, in der Perilymphe und gelangen so zu den Haarzellen. Darüber hinaus steht das Perilymphe-System auch mit dem Labyrinth in Verbindung. Dadurch gelangen Medikamente aus der Perilymphe der Schnecke auch in die Perilymphe des Labyrinths, also des Gleichgewichtsorgans.

Somit sind alle pharmazeutischen Formulierungen bevorzugt, welche geeignet sind, den Wirkstoff lokal an der Rundfenstermembran zu applizieren. Ferner bevorzugt ist, wenn die pharmazeutischen Formulierungen einen Membranpenetrationsförderer enthalten, der den Durchtritt des ß-Carbolins durch die Rundfenstermembran unterstützt. Dementsprechend sind flüssige oder gelförmige Formulierungen insbesondere bevorzugt. Natürlich ist es auch möglich, den Wirkstoff oral zu applizieren.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Pharmazeutische Zusammensetzungen für eine beliebige Art der Verabreichung von ß-Carbolinen enthalten eine für den therapeutischen Effekt ausreichende Menge an ß-Carbolin und, falls nötig, anorganische oder organische, feste oder flüssige pharmazeutisch verträgliche Trägerstoffe. Pharmazeutische Zusammensetzungen, die für eine topische Verabreichung im Mittelohr geeignet sind, beinhalten wässrige Lösungen oder Suspensionen, die vor der Verabreichung im Mittelohr hergestellt werden können, z. B. im Fall von lyophilisierten Formulierungen, die ß-Carboline alleine oder zusammen mit einem Träger enthalten. Die pharmazeutischen Zusammensetzungen schließen weiterhin Gele, die biologische abbaubar oder nichtbiologisch abbaubar, wässrig oder nicht-wässrig oder Mikrosphären basiert sind. Beispiele für solche Gele beinhalten Polyoxamere, Hyaluronate, Xyloglucane, Chitosane, Polyester, Polylactide, Polyglycolide oder deren Co-Polymer PLGA, Saccharoseacetatisobutyrat und Gylcerinmonooleat. Pharmazeutische Zusammensetzungen, die für eine enterale oder parenterale Verabreichung geeignet sind, beinhalten Tabletten oder Gelatinekapseln oder wässrige Lösungen oder Suspension wie oben beschrieben.

Die pharmazeutischen Zusammensetzungen können sterilisiert werden und/oder Adjuvantien enthalten, z. B. Konservierungsstoffe, Stabilisatoren, Feuchthaltemittel und/oder Emulgatoren, Salze für die Regulierung des osmotischen Drucks und/oder Puffer. Die erfindungsgemäße pharmazeutische Zusammensetzung kann, falls gewünscht, weitere Wirkstoffe enthalten. Diese pharmazeutischen Zusammensetzungen können durch eine beliebige Methode, die aus dem Stand der Technik bekannt ist, hergestellt werden, z. B. durch herkömmliche Methoden wie Vermischung, Granulierung, Konfektionierung, Lösung und Lyophilisierung und können von ungefähr 0,01 bis 100 Prozent, bevorzugt zwischen 0,1 und 50 Prozent, bei Lyophilisaten bis zu 100 Prozent an ß-Carbolin enthalten.

Bei einer bevorzugten Ausführungsform wird die erfindungsgemäße pharmazeutische Zusammensetzung für eine topische Verabreichung formuliert. Geeignete Träger für eine otogene Verabreichung sind organische oder anorganische Substanzen, die pharmazeutisch verträglich sind und die nicht mit ß-Carbolinen und/oder den weiteren Wirkstoffen reagieren, z. B. Kochsalz, Alkohole, pflanzliche Öle, Benzylalkohole, Alkylglycole, Polyethylenglycole, Glycerintriacetate, Gelatine, Kohlenhydrate wie Lactose oder Stärke, Magnesiumcarbonat (Magnesia, *Chalk*), Stearat (Wachse), Talk und Petrolatum (Vaseline). Die beschriebenen Zusammensetzungen können sterilisiert werden und/oder Hilfsstoffe wie Gleitmittel, Konservierungsstoffe wie Thiomersal, z. B. 50 Gewichtsprozent), Stabilisatoren und/oder Feuchthaltemittel, Emulgatoren, Salze zur Beeinflussung des osmotischen Drucks, Puffersubstanzen, Farbstoffe und/oder Geschmacksstoffe enthalten. Diese Zusammensetzungen können gegebenenfalls auch einen oder mehrere weitere Wirkstoffe enthalten. Erfindungsgemäße otogene Zusammensetzungen können verschiedene Stoffe und/oder Substanzen umfassen, beinhaltend andere biologische aktive Substanzen wie Antibiotika, antiinflammatorische Wirkstoffe wie Steroide, Cortisone, Analgetika, Antipyrine, Benzocaine, Procaine usw.

Erfindungsgemäße Zusammensetzungen für die topische Verabreichung können andere pharmazeutische verträgliche Stoffe und/oder Substanzen enthalten. In bevorzugten Ausführungsformen der vorliegenden Erfindung wird ein topischer Exzipient ausgewählt, der die Abgabe von ß-Carbolinen und des gegebenenfalls weiteren Wirkstoffs oder der Wirkstoffe an das Blutkreislaufsystem oder an das Zentralnervensystem nicht verstärkt, wenn dieser am Ohr, im Ohr oder im Gehörgang verbreicht wird. Zum Beispiel wird üblicherweise bevorzugt, dass der topische Exzipient keine wesentlichen Ausschlußeigenschaften aufweist, die eine perkutane Übertragung durch die Mucosa in das systemische Kreislaufsystem verstärken. Solche Trägerstoffe beinhalten Hydrocarbonsäuren, wasserfreie Absorptionsmittel wie hydrophiles Petrolatum (Vaseline) and wasserfreies Lanolin (z. B. Aquaphor) und Mittel auf Basis von Wasser-Öl-Emulsionen wie Lanolin und *Cold Cream.* Mehr bevorzugt sind Trägerstoffe, die im wesentlichen nicht-ausschließend sind und beinhalten üblicherweise diejenigen Trägerstoffe, die wasserlöslich sind, wie auch Stoffe auf Basis von Öl-in-Wasser-Emulsionen (Cremes oder hydrophile Salben) und Stoffe auf wasserlöslicher Basis wie auf Polyethylenglycol basierende Trägerstoffe and wässrige Lösungen, die mit verschiedenen Stoffen wie Methylcellulose, Hydroxyethylcellulose und Hydroxpropylmethylcellulose geliert wurden.

Bei oraler Verabreichung in Form von Tabletten oder Kapseln können die erfindungsgemäßen ß-Carboline mit nicht-toxischen pharmazeutisch verträglichen Hilfsstoffen ausgewählt aus der Liste umfassend Bindemittel wie vorgelierte Maisstärke, Polyvinylpyrrolidon oder Hydroxypropylmethylcellulose; Füllstoffe wie Lactose, Saccharose, Glucose, Mannitol, Sorbitol und andere reduzierende und nicht-reduzierende Zucker, mikrokristalline Cellulose, Calciumsulfat oder Calciumhydrogenphosphat; Gleitmittel wie Magnesiumstearat, Talkum oder Silica, Stearinsäure, Natriumstearylfumarat, Glycerylbehenat, Calciumstearat und ähnliche; Zerfallsbescheluniger wie Kartoffelstärke oder Natriumglycolat-Stärke; Befeuchtungsmittel wie Natriumdodecylsulfat; Farbstoffe; Geschmacksstoffe; Gelatine; Süßstoffe; natürliche und syntheische Gummis wie Gummi arabicum, Tragacanth oder Alginate; Puffersalze; Carboxymethylcellulose; Polyethylenglycol; Wachse und ähnliche.

Die Tabletten können mit einer konzentrierten Zuckerlösung umhüllt sein, die zum Beispiel Gummi arabicum, Gelatine, Talcum, Titandioxid und ähnliches enthalten kann. Bei einer anderen Ausführungsform können die Tabletten mit einem Polymer umhüllt sein, das sich in einem leichtflüchtigem organischem Lösungsmittel oder einer Mixtur an organischen Lösungsmitteln löst. In bevorzugten Ausführungsformen sind die erfindungsgemäßen ß-Carboline als Tabletten zur sofortigen Freigabe oder als Tabletten in Retardform formuliert. Dosierungsformen zur sofortigen Freigabe erlauben die Freisetzung eines Großteils oder der Gesamtmenge an ß-Carobolinen innerhalb einer kurzen Zeitspanne wie 60 Minuten oder weniger und ermöglichen die rasche Absorption der ß-Carboline. Retardformen für die oralen Dosierungsformen erlauben dje verzögerte Freisetzung über einen längeren Zeitraum, um einen therapeutisch wirksamen Plasmaspiegel an ß-Carbolinen zu erreichen und/oder diesen therapeutisch wirksamen Plasmaspiegel über einen längeren Zeitraum stabil zu halten und/oder andere pharmakokinetische Eigenschaften der ß-Carboline zu modifizieren.

Zur Formulierung von Weichgelatinekapseln können die erfindungsgemäßen ß-Carboline zum Beispiel mit einem pflanzlichen Öl oder Polyethylenglycol vermengt werden. Hartgelatinekapseln können die ß-Carboline in Granulatform enthalten unter Verwendung von entweder der obengenannten Hilfsstoffe für Tabletten wie Lactose, Saccharose, Mannitol, Stärken wie Kartoffelstärke, Maisstärke oder Amylopectin, Cellulosederivate oder Gelatine. Auch flüssige oder semiflüssige Formen der erfindungsgemäßen ß-Carboline können in die Hartgelatinekapseln gefüllt werden.

Die erfindungsgemäßen ß-Carboline können auch in Mikrokügelchen oder Mikrokapseln, die z. B. aus Polyglycolsäure/Milchsäure (PGLA) hergestellt sind, eingebracht werden. Bioverträgliche Polymere, ausgewählt aus der Liste umfassend Polymilchsäure, Polyglycolsäure, Copolymere von Polymilchsäure und Polyglycolsäure, Poly-ε-Caprolacton, Polyhydroxbuttersäure, Polyoethoester, Polyacetale, Polyhydropurane, Polycyanoacrylate und vernetzte pder amphiphatische Blockcopolymere von Hydrogelen, können verwendet werden, um eine kontrollierte Freisetzung der erfindungsgemäßen ß-Carboline aus den pharmazeutischen Zusammensetzungen der vorliegenden Erfindung zu erreichen.

In einer weiteren Ausführungsform der Erfindung werden die ß-Carboline als eine orale flüssige Formulierung bereitgestellt. Flüssige Zubereitungen für die orale Verabreichung können in Form von Lösungen, Sirupen, Emulsionen oder Suspensionen bereitgestellt werden. Alternativ können die oralen flüssigen Formulierungen durch Rekonstitution der trockenen oralen Formulierung mit Wasser oder einem anderen geeignetem Trägerstoff vor der Anwendung hergestellt werden. Zuberetungen zur oralen Verabreichung können geeignet formuliert werden, so dass eine kontrollierte oder retardierte Freisetzung der erfindungsgemäßen ß-Carboline und gegebenfalls weiterer Wirkstoffe erreicht werden kann.

Bei oraler Verabreichung in flüssiger Form können die erfindungsgemäßen ß-Carboline mit nicht-toxischen pharmazeutisch verträglichen inerten Trägerstoffen wie Ethanol, Glycerin, Wasser; Suspensionsmittel wie Sorbitolsyrup, Cellulosederivate oder gehärteten essbaren Fetten; Eulgatoren wie Lecithin oder Gummi arabicum; nicht wässrigen Trägerstoffen wie Mandelöl, ölige Ester, Ethanol oder fraktionierte pflanzliche Öle; Konservierungstoffen wie Methyl- oder Propyl-p-hydroxybenzoate oder Sorbinsäure; und ähnlichen. Stabilisatoren wie z. B. Antioxidantien wie Butylhydroxyanisol, Butylhydroxytoluol, Propylgallat, Natriumascorbat, Zitronensäure können auch zur Stabilisierung der Dosierungsform verwendet werden. Zum Beispiel können die Lösungen ungefähr 0.2 Gewichtprozent bis ungefähr 20 Gewichtprozent an ß-Carbolinen enthalten, wobei die Ausgleichsmasse Zucker und eine Mischung aus Ethanol, Wasser, Glycerin und Propylenglycol ist. Optional können diese flüssigen Formulierungen Farbstoffe, Geschmacksstoffe, Saccharin, Carboxylcellulose als Verdickungsmittel und/oder andere Hilfsstoffe enthalten.

Bei einer weiteren Ausführungsform wird eine therapeutische wirksame Dosis an erfindungsgemäßen ß-Carbolinen über eine Lösung oral verabreicht, wobei die Lösung einen Konservierungsstoff, Süßstoff, Lösungsvermittler und ein Lösungsmittel enthält. Die Lösung zur oralen Verabreichung kann einen oder mehrere Puffer, Geschmackstoffe oder weitere Exzipienten enthalten. Bei einer weiteren Ausführungsform wird Pfefferminze oder ein anderer Geschmacksstoff zu der Lösung der erfindungsgemäßen ß-Carbolinen für die orale Verabreichung hinzugefügt.

Für die Verabreichung über Inhalierung können die erfindungsgemäßen ß-Carboline in geeigneter Weise verabreicht werden wie in der Darreichungsform als Aerosolspray mit unter Druck stehenden Packungen oder einem Zerstäuber, unter Verwendung eines geeigneten Treibgases wie Dichlorfluoromethan, Trichlorfluoromethan, Dichlortetrafluroethan, Kohlendioxid oder einem anderen geeignetem Gas. Bei Verwendung eines unter Druck stehenden Aerosols kann die Dosis dadurch bestimmt werden, dass ein Ventil bereitgestellt wird, um eine abgemessene Menge zu verabreichen. Kapseln und Kartuschen aus z. B. Gelatine für die Verwendung in Inhalatoren or Insufflatoren können so formuliert werden, dass die Kapseln und Kartuschen eine Pulvermischung der erfindungsgemäßen ß-Carbolinen und gegebenenfalls eines weiteren oder mehrerer Wirksotffe und eine geeignete Pulverbasissubstanz wie Lactose oder Stärke enthalten.

Lösungen für die parenterale Verabreichung durch Injektion können aus einer wässrigen Lösung eines wasserlösöiche pharmazeutisch verträglichen Salzes der erfindungsgemäßen ß-Carboline mit einer Konzentration von ungefähr 0,5 Gewichtprozent bis ungefähr 10 Gewichtprozent hergestellt werden. Diese Lösungen können auch Stabilisatoren und/oder Puffersubstanzen enthalten und können in geeigneter Weise über Ampullen mit verschiedenen Dosiseinheiten bereit gestellt werden.

Somit sind die hier vorgestellten Verbindungen nützlich für die Herstellung von pharmazeutischen Formulierungen zur Prophylaxe und/oder Behandlung von von akuten und chronischen Ohrenerkrankungen und Hörschäden, Schwindel und Gleichgewichtsstörungen insbesondere von Hörsturz, Knalltrauma, Innenohrschwerhörigkeit durch chronische Lärmexposition, Altersschwerhörigkeit, Trauma während der Implantation von Innenohrprothesen (Insertionstrauma), Schwindel aufgrund von Erkrankungen im Innenohrbereich, Schwindel im Zusammenhang und/oder als Symptom der Menière'schen Erkrankung, Gleichgewichtsstörungen im Zusammenhang und/oder als Symptom der Menière'schen Erkrankung, Tinnitus und Hörschäden aufgrund von Antibiotika und Zytostatika.

### Beschreibung der Figuren

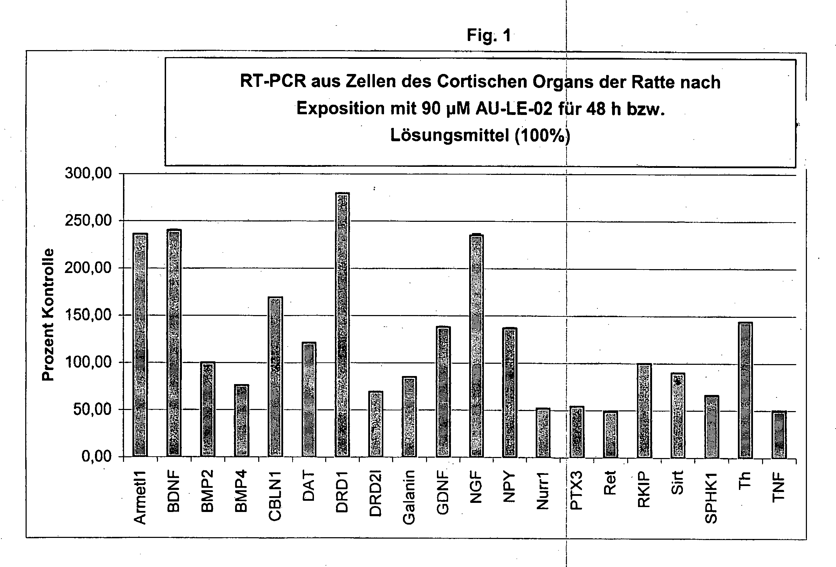

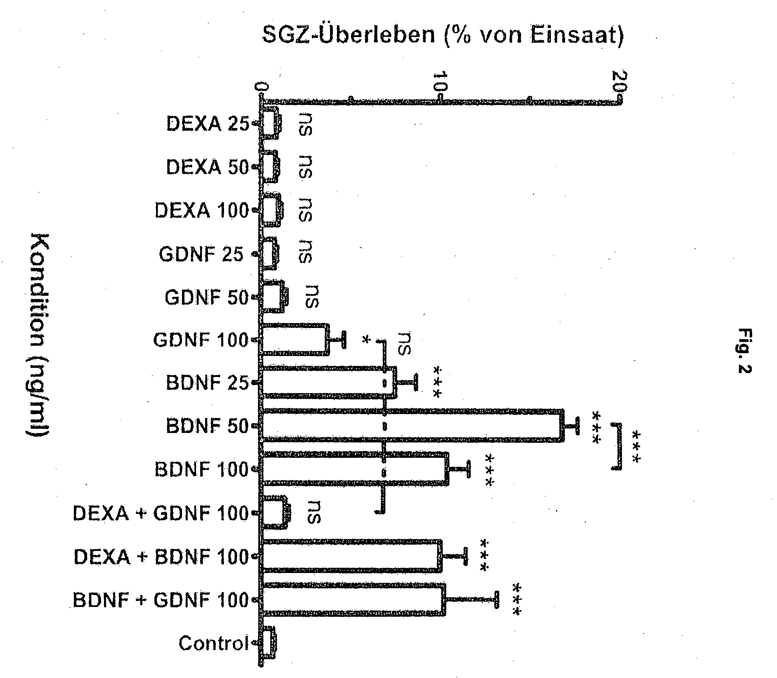

- Fig. 1: zeigt eine graphische Auswertung der quantitativen RT-PCR (reverse Transkriptase Polymerase-Kettenreaktion) von Innenohrzellen (Cortisches Organ der Cochlea) der Ratte nach Exposition mit 90 µM 9-Methyl-β-Carbolin (9-Me-BC) für 48h bzw. Lösungsmittel (100%). Die Werte über 100 % bedeuten, dass 9-Me-BC die Transkription des betreffenden Gens während der Inkubation aktiviert hat, während für Werte unter 100 % das Gegenteil gilt.
Die in Fig. 1 verwendeten Abkürzungen haben folgende Bedeutung:
- Armetl1:: konservierter Dopamin neurotropher Faktor
- BDNF:: Brain-derived neurotropher Faktor
- BMP2:: Bone morphogenetic Protein 2
- Cbln1:: Cerebellin 1 precursor Protein
- DAT:: Dopamintransporter
- DEXA:: Dexamethason
- DRD1:: Dopaminrezeptor Subtyp 1
- DRD21:: Lange Variante des Dopaminrezeptors Subtyp 2
- GDNF:: Glial cell line derived neurotropher Faktor
- NGF:: Nervenwachstumsfaktor (nerve growth factor)
- NPY:: Neuropeptid Y
- Nurr 1:: Nuclear receptor regulated 1 Protein
- PTX:: Paired-like homeodomain Transcriptionsfaktor
- Ret:: Rearranged during transfection Rezeptor
- RKIP:: Raf-1 Kinase Inhibitor Protein
- Sirt:: silent information regulator
- Th:: Tyrosinhydroxylase
- TNF:: Tumornekrosefaktor
- Fig. 2: zeigt eine graphische Auswertung der Effekte von verschiedenen Konzentrationen und Kombinationen von Glial cell line derived neurotrophem Faktor (GDNF), brain-derived neurotrophem Faktor (BDNF) und Dexamethason (DEXA) auf das Überleben der Zellen des Spiralganglions des Innenohrs nach 48 Stunden in Kultur. Die Höhe der Säulen gibt den Mittelwert ± Standardfehler an und repräsentiert 24-32 Beobachtungen aus drei oder vier verschiedenen Experimenten. Die Signifikanzen der verschiedenen Vergleiche mit der Kontrollgruppe sind über den Balken dargestellt, während andere Vergleiche separat mittels Klammern verdeutlicht sind (P<0,05 *; P< 0,01 **; P<0,001 ***). Aus: Medizinische Hochschule Hannover Forschungsbericht 2005, Klinik Für Hals-Nasen-Ohrenkrankheiten (Direktor Th. Lenarz), Seite 475.

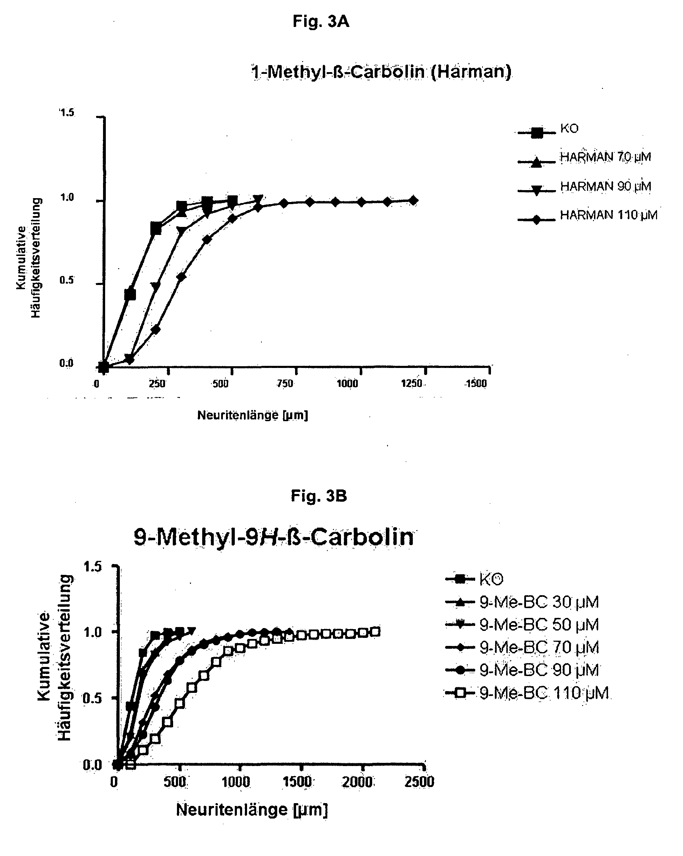

- Fig. 3A: zeigt die Neuritenlänge von humanen SH-SY5Y Zellen, die wie oben beschrieben mit Harman 14 Tage lang in Kultur behandelt wurden. Der Unterschied zwischen der Kontrollgruppe und der Gruppe, die mit 110 µM Harman behandelt wurde, war signifjkant (P<0,001). Harman führt also zu einer Zunahme der Neuritenlänge.
- Fig. 3B: zeigt die Neuritenlänge von humanen SH-SY5Y Zellen, die wie oben beschrieben mit 9-Methyl-9H-ß-Carbolin 14 Tage lang behandelt wurden. Der Unterschied zwischen der Kontrollgruppe und den Gruppen, die mit 70, 90 oder 110 µM 9-Methyl-9H-ß-Carbolin behandelt wurden, war signifjkant (P<0,001). 9-Methyl-9H-ß-Carbolin führt also zu einer Zunahme der Neuritenlänge.

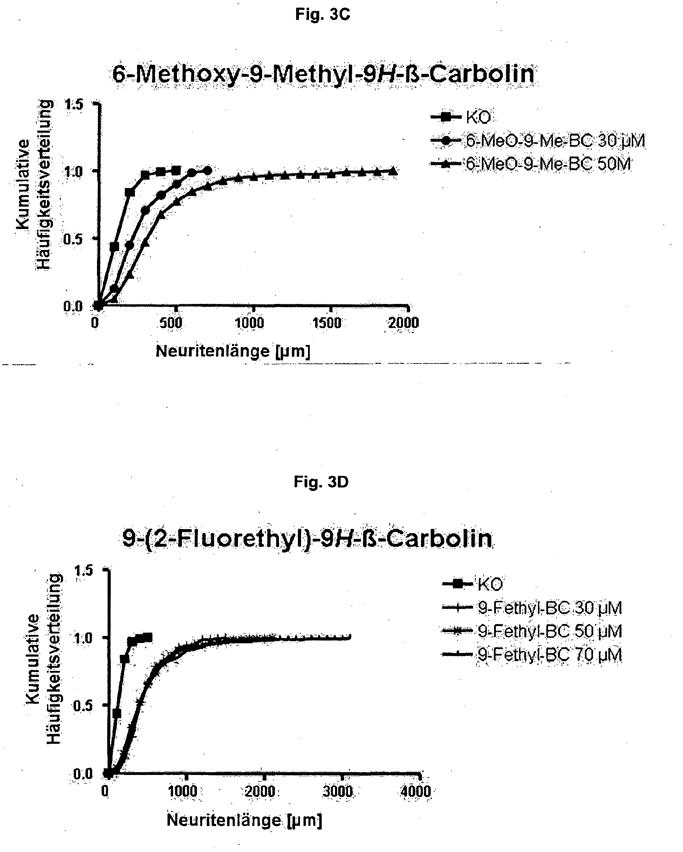

- Fig. 3C: zeigt die Neuritenlänge von humanen SH-SY5Y Zellen, die wie oben beschrieben mit 6-Methoxy-9-methyl-9H-ß-Carbolin behandelt wurden. Der Unterschied zwischen der Kontrollgruppe und der Gruppe, die mit 50 µM 6-Methoxy-9-methyl-9H-ß-Carbolin behandelt wurden, war signifjkant (P<0,001). 6-Methoxy-9-methyl-9H-ß-Carbolin führt also zu einer Zunahme der Neuritenlänge.
- Fig. 3D: zeigt die Neuritenlänge von humanen SH-SY5Y Zellen, die wie oben beschrieben mit 9-(2-Fluorethyl)-9H-ß-Carbolin behandelt wurden. Der Unterschied zwischen der Kontrollgruppe und den Gruppen, die mit 30, 50 oder 70 µM 9-(2-Fluorethyl)-9H-ß-Carbolin behandelt wurden, war signifjkant (P<0,001). 9-(2-Fluorethyl)-9H-ß-Carbolin führt also zu einer Zunahme der Neuritenlänge.

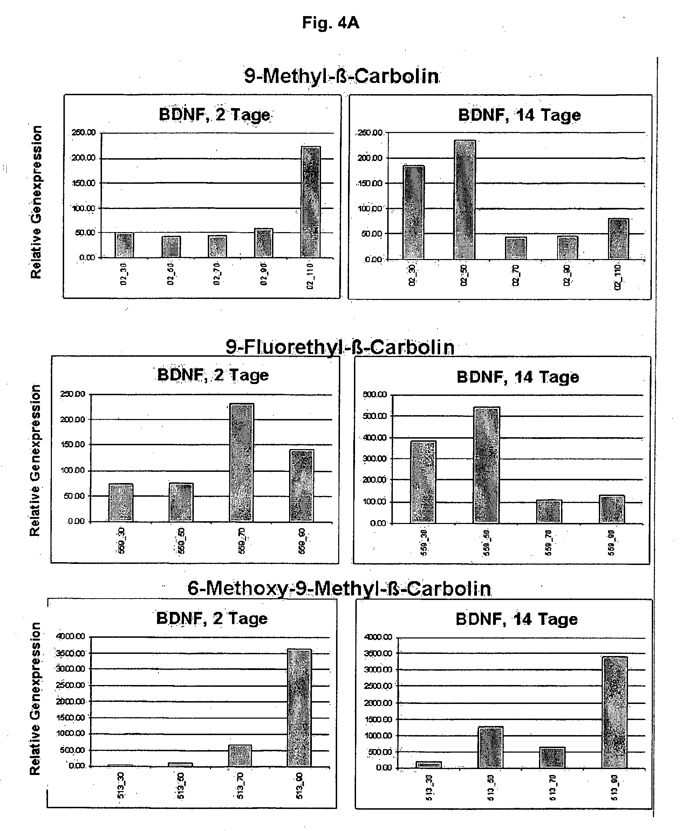

- Fig. 4A: zeigt die relative Genexpression von BDNF (brain-derived neurotrophic factor) in humanen SH-SY5Y Zellen. Die Transkription von BDNF war nach zwei Tagen nur durch die höchste Konzentration (110 µM) von LE-02 (9-Methyl-ß-Carbolin) stimuliert, während nach 14tägiger Exposition die niedrigen Konzentrationen von 30 und 50 µM eine Stimulierung induzierten. Eine solche Verschiebung wurde auch für 9-Fluorethyl-ß-Carbolin (Substanz Nr. 559 in Fig. 4A; nach 2 Tagen: Stimulierung durch 70 und 90 µM; nach 14 Tage durch 30 und 50 µM) gefunden. Im Gegensatz dazu führte 6-Methoxy-9-Methyl-ß-Carbolin (Substanz Nr. 513 in Fig. 4A) zu einem enormen, dosisabhängigen Anstieg der BDNF Transkription.

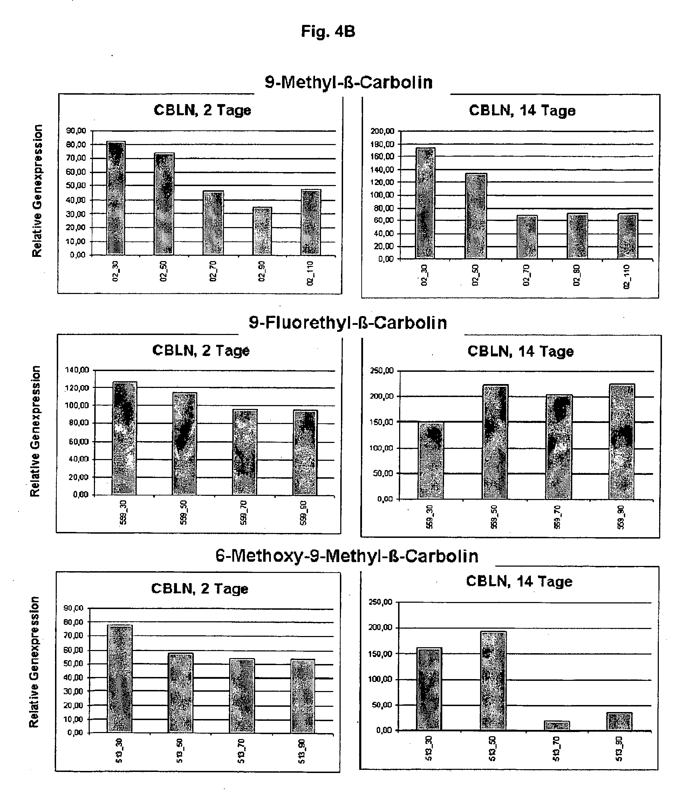

- Fig.4B: zeigt die relative Genexpression von Cerebellin-1 Precursor Protein (CBLN). Cerebellin-1 Precursor Protein war nach zweitägiger Exposition nur durch 9-Fluorethyl-ß-Carbolin erhöht. Nach vierzehntägiger Exposition erhöhten alle getesteten ß-Carboline die CBLN Expression. Niedrige Konzentrationen der Testsubstanzen wirkten stärker als hohe Konzentrationen mit Ausnahme von 9-Fluorethyl-ß-carbolin, von dem alle untersuchten Konzentrationen etwa gleich stark stimulierten.

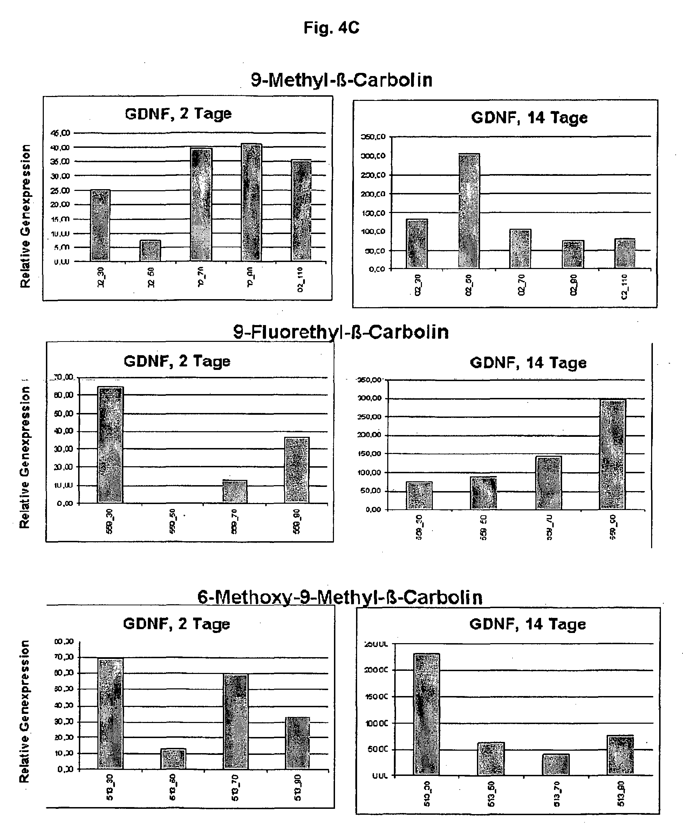

- Fig. 4C: zeigt die relative Genexpression von GDNF (glial cell line-derived neurotrophic factor). Die vierzehntägige Exposition führte für einzelne Konzentrationen zu einer bis zu dreifachen Stimulierung.

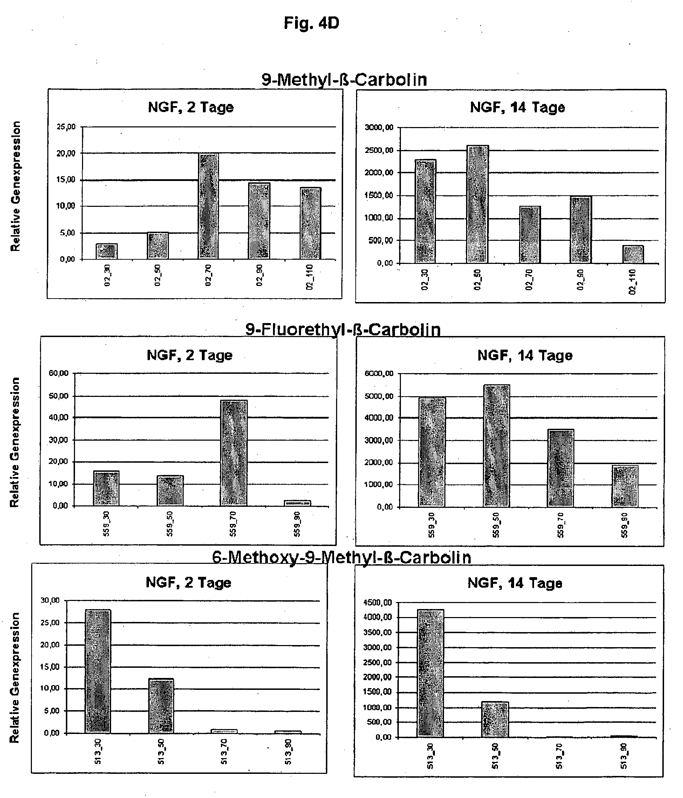

- Fig. 4D: zeigt die relative Genexpression von NGF (nerve growth factor). Nach vierzehntägiger Exposition war die Genexpression 20 bis 50-fach erhöht. Auch für NGF gilt, dass die niedrigen Konzentrationen wesentlich wirksamer waren.

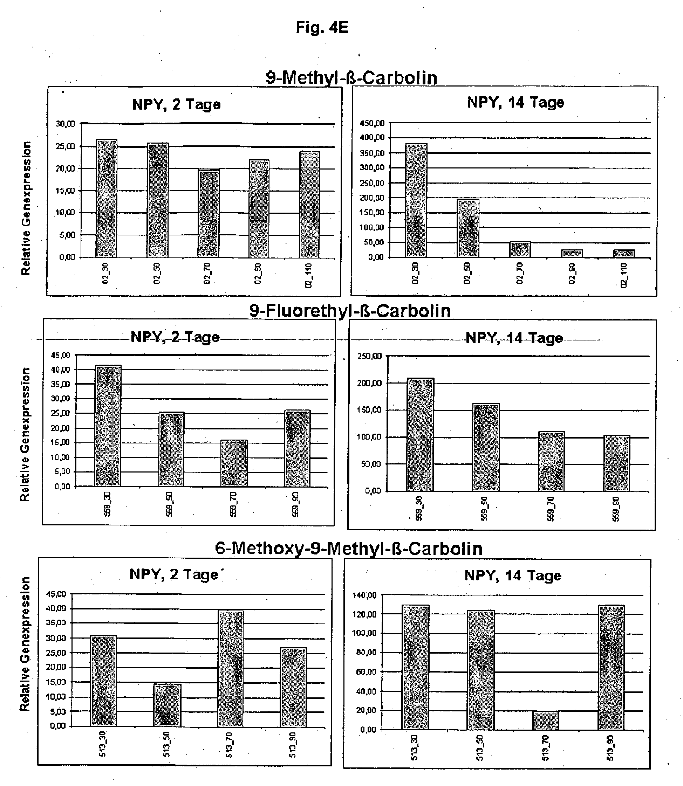

- Fig. 4E: zeigt die relative Genexpression von NPY (Neuropeptid Y). Nach vierzehntägiger Exposition war die Genexpression zwischen 1,3 und 4-fach verstärkt. Für 9-Methyl-ß-Carbolin und 9-Fluorethyl-ß-Carbolin waren die niedrigen Konzentrationen wirksamer als die höheren.

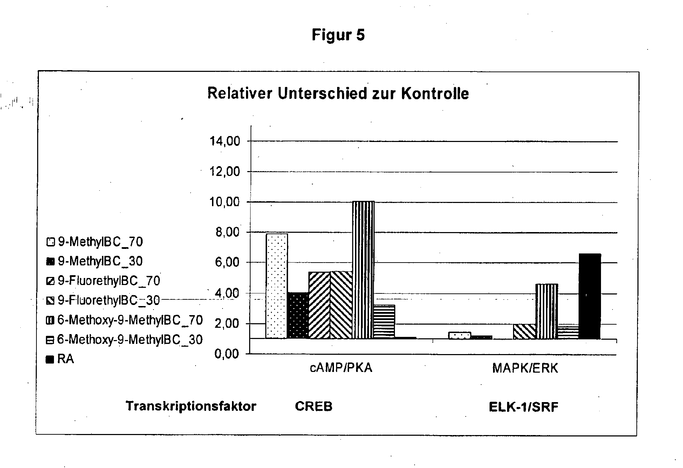

- Fig. 5: zeigt, dass die 9-Methyl-ß-Carbolin, 9-Fluorethyl-ß-Carbolin und 6-Methoxy-9-Methyl-ß-Carbolin dosisabhängig die Bildung des Transkriptionsfaktors CREB bis zum 10fachen der Kontrollzellen in SH-SY5Y Zellen aktivieren (drei unabhängige Experimente, 48 h Inkubation).

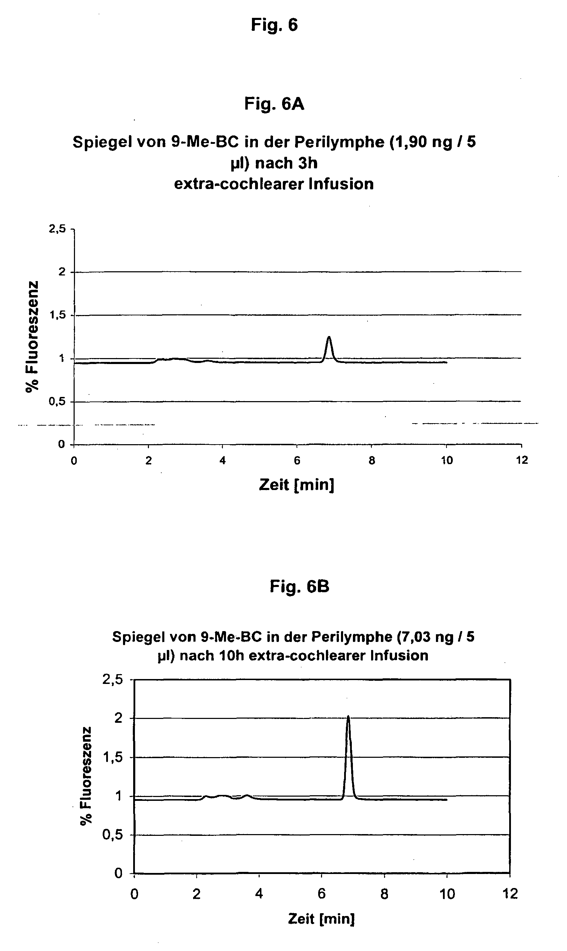

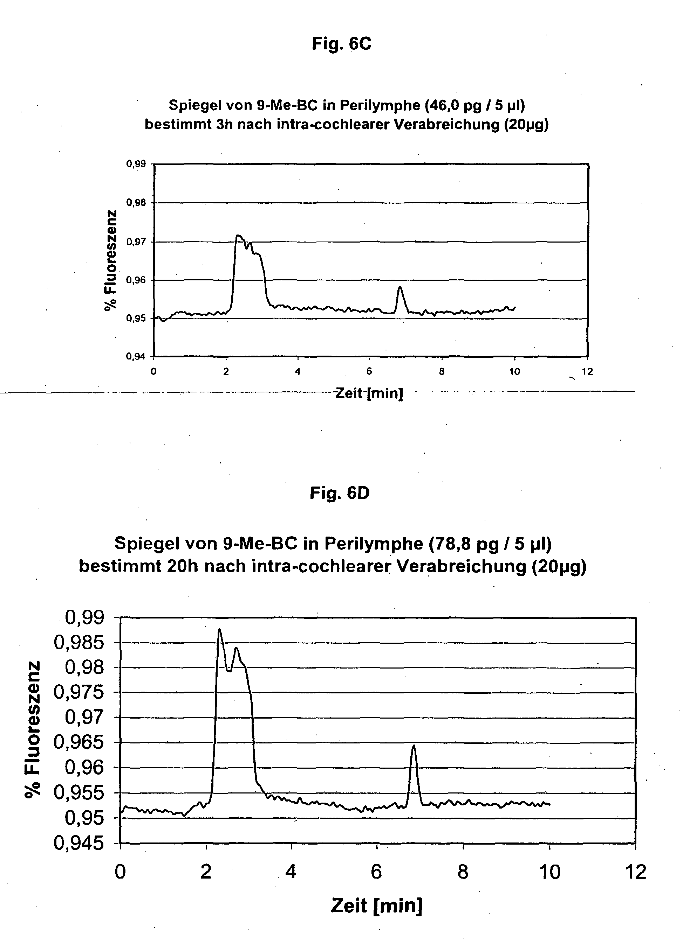

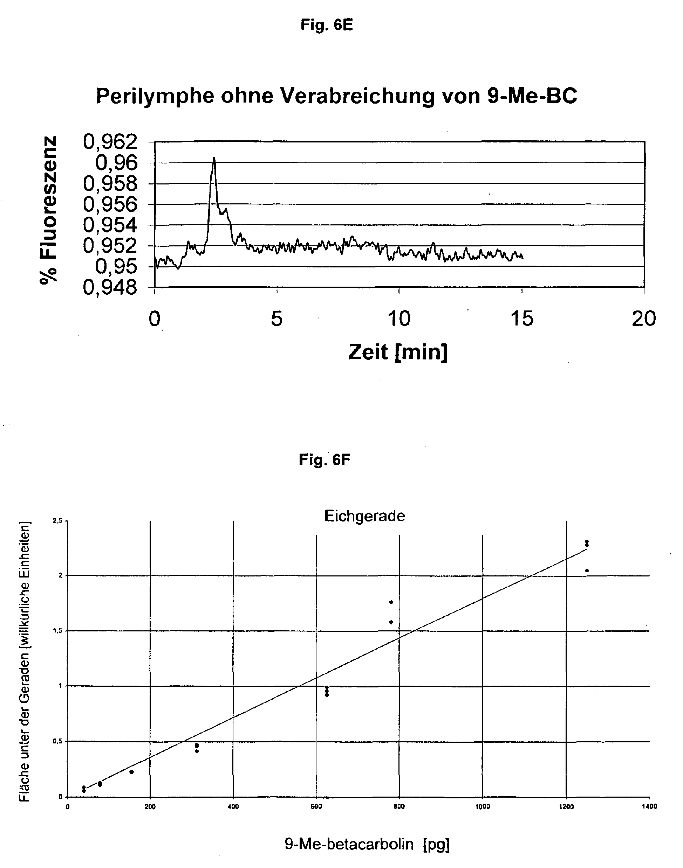

- Fig. 6: die Figuren 6A bis 6F zeigen Chromatogramme der Perilymphe, die aus den Experimenten in Beispiel 29 gewonnen wurden. Die Chromatogramme gemäß der Figuren 6A und 6B wurden aufgenommen mit Perilymphe, die nach 3-stündiger (Fig. 6A) bzw. 10-stündiger (Fig. 6B) Infusion von 9-Methyl-ß-Carbolin (9-Me-BC) in den Mittelohrbereich von Meerschweinchen gewonnen wurde. Die Chromatogramme gemäß der Figuren 6C und 6D wurden aufgenommen mit Perilymphe, die nach 3-stündiger bzw. 20-stündiger Injektion von 9-Methyl-ß-Carbolin durch das runde Fenster direkt in die Cochlea von Meerschweinchen gewonnen wurde. Das Chromatogramm gemäß der Figur 6E wurde aufgenommen mit Perilymphe, die keiner Behandlung mit 9-Methyl-ß-Carbolin ausgesetzt war. Das Chromatogramm gemäß der Figur 6F gibt eine Eichgerade wieder, in der die Konzentration von 9-Methyl-ß-Carbolin einer Fläche unter dem dafür erhaltenen chromatographischen Maximum zugeordnet wurde.

### Beispiele

### Beispiel 1: Synthese von 9-Methyl-ß-Carbolin

### Syntheseschema für 9-Methyl-ß-Carbolin

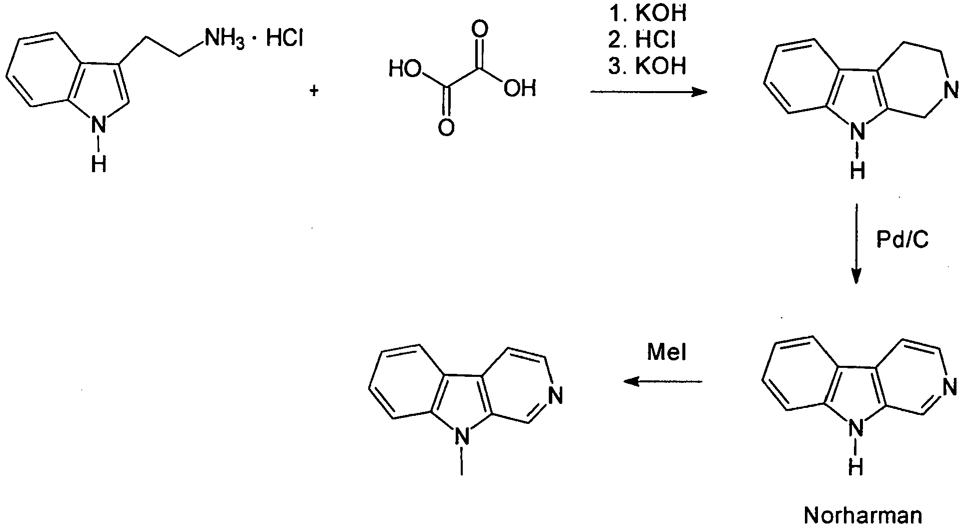

Eine gerührte Lösung von 13 g (0,0756 Mol) 1,2,3,4-Tetrahydro-ß-Carbolin, hergestellt aus Tryptaminhydrochlorid und Glyoxalsäure, wie von Ho et al. (Ho BT, Mclsaac WM, Walker KE, Estevez V, Inhibitors of monoamine oxidase. Influence of methyl substitution on the inhibitory activity of beta-carbolines, J Pharm Sci 57: 269, 1968) beschrieben und 2,6 g Pd/C (10%) in 600 ml Cumol wurden unter Stickstoffatmosphäre für 90 min refluxiert. Nach Zugabe von 100 ml Ethanol wurde die heiße Lösung filtriert und die Kohle mit 3 x 30 ml heißem Ethanol extrahiert. Die vereinigten flüssigen Fraktionen wurden eingeengt und der Rückstand aus Toluol kristallisiert, um 10,5 g (82%) Norharman zu erhalten. Die Methylierung in 9-Position wurde, wie in der Literatur (Ho et al. J Pharm Sci 1968) beschrieben, doch mit einer verbesserten Aufarbeitung, durchgeführt: 1 g (5,95 mmol) Norharman wurden in 10 ml trockenem DMF unter Stickstoffatmosphäre gelöst. Danach wurden 0,36 g (14,9 mmol) Natriumhydrid als eine 60%ige Dispersion in Petroleum bei 0°C hinzugefügt. Nach Abkühlen der Reaktionsmischung auf Raumtemperatur wurde diese auf -10°C abgekühlt und es wurden 0,84 g (5,95 mmol) Methyliodid hinzugefügt. Nach weiterem Rühren für 12 h ließ man die Mischung erneut auf Raumtemperatur abkühlen. Alle flüchtigen Bestandteile wurden unter reduziertem Druck entfernt. Danach wurden 100 ml Wasser hinzugefügt und die Mischung wurde mit 3 x 50 ml CHCl₃ extrahiert. Die vereinigten organischen Fraktionen wurden mit 5 x 20 ml Wasser gewaschen und zur Trockne eingedampft. Der Rückstand wurde in 100 ml 2N Salzsäure aufgenommen. Um das Edukt vom gewünschten methylierten Produkt abzutrennen, wurde eine lonenpaar-Extraktion des HCl-Salzes in CHCl₃ und in einem Flüssig/Flüssig-Extraktor für 2 Tage durchgeführt. Nach Entfernen des Lösungsmittels erhielt man 0.7 g (64%) gelbe Kristalle von 9-Methyl-ß-Carboliniumhydrochlorid.

Schmpkt: 295° ; GC/MS für die freie Base: m/z = 182 (100%), 167 (5%), 140 (10%), 127 (10%), 113 (5%), 91 (10%). ¹H-NMR (HCl-Salz): δ (ppm) Methanol *d4*, 250 MHz: 4.06 s, 3H, N-CH₃; 7.28 - 7.35, dt, *J* = 1.2; 6.8, 1 H, H6; 7.58 - 7.70, m, 2H, H7, H8; 8.13 - 8.16, d, *J* = 5.4, 1 H, H4; 8.18 - 8.21, d, *J* = 7.9, 1 H H5; 8.31 - 8.33, d, *J* = 5.4, 1 H, H3; 8.89, s, 1H, H1.

### Beispiele 2-13: Herstellung der Verbindungen A bis L

Die Synthese der Verbindungen A bis L erfolgt gemäß Beispiel 1, indem die entsprechenden Alkyliodide, Alkylbromide oder Alkyltosylate eingesetzt wurden. Die Ausbeuten lagen zwischen 30% und 75% der Theorie.

### Beispiel 14: Untersuchungen an Innenohrzellen (Cochlea) der Ratte

Zum Nachweis der Wirksamkeit der erfindungsgemäßen ß-Carboline wurden Versuche durchgeführt, um festzustellen, welche Auswirkungen diese Verbindungsklasse auf Ohrenerkrankungen, Hörschäden und Schwindel hat. Als Beispielsubstanz wurde 9-Methyl-ß-Carbolin (9-Me-BC, VI) ausgewählt. Die Wirksamkeiten der anderen getesteten Substanzen werden in Relation zur Wirksamkeit von 9-Methyl-ß-Carbolin (9-Me-BC, VI) angegeben.

Zunächst wurden Organkulturen der Cortischen Organe der Cochlea von Ratten hergestellt, welche im nächsten Schritt mit 9-Methyl-ß-Carbolin in Kontakt gebracht wurden. Als Marker für die Wirkung des 9-Methyl-ß-Carbolin auf die Cortischen Organe wurde die Veränderung der Expression verschiedener Neurotrophine gemessen. Dazu wurde die RNS aus dem Cortischen Gewebe gewonnen, in cDNS umgeschrieben und die Konzentrationen der cDNS der Neurotrophine mit Hilfe der real-time RT-PCR Methode bestimmt.

Die Cortischen Organe der Cochlea wurden von ca. 40 Ratten am Tag 4 post partum in mehreren Sitzungen präpariert. Anschließend wurde die Organkultur entweder einer Konzentration von 90 µM 9-Me-BC für 48 Stunden ausgesetzt oder einer entsprechenden Menge an Lösungsmittel (Kontrollbedingung). Danach wurde die Organkultur mit PBS-EDTA gewaschen (PBS-EDTA der Firma Biochrom verdünnt 1:27 mit PBS). Anschließend wurde die Probe bei -80 °C eingefroren. Die Gesamt-Ribonukleinsäure (RNS) wurde mittels RNeasy Lipid Tissue Mini Kit der Firma Qiagen, Hilden, isoliert. Mit der Probe der isolierten RNS wurde ein DNase Verdau durchgeführt mit anschließenden mehrfachen Waschschritten entsprechend den Angaben des Herstellers des Kits. Die RNS wurde dann mit RNase-freiem Wasser eluiert. Die Menge und Qualität der RNS wurden photometrisch bestimmt. 1 µg RNS jeder Probe wurde in die komplementäre DNS (cDNS) überschrieben unter Verwendung des Kits der Firma Roche Applied Science, Mannheim. Die Echtzeit reverse Transkriptase Polymerasekettenreaktion (real-time RT-PCR) wurde unter Verwendung der Fluoreszenz Resonanz Energietransfer (FRET) Methode mit dem LightCycler System der Firma Roche Applied Science, Mannheim, durchgeführt. Dabei wurde der LightCycler FastStart DNA Master hybridization probes Kit derselben Firma verwendet. Das Hydroxymethylbilansynthase Gen (HMBS) wurde als sogenanntes housekeeping Gen für die relative Quantifizierung ausgewählt. Geeignete Primer und Proben wurden von der Firma Tib Molbiol, Berlin, entworfen und synthetisiert. Zu jeder Reaktion wurden 25 ng cDNS zugegeben. Die Bedingungen der Zyklen waren wie folgt: Denaturierung bei 94°C für 5 Min, 55 Zyklen von 7 Sec unter Denaturierungsbedingungen, Bindung der Primer und der Probe bei der geeigneten Temperatur für 10 Sec und Verlängerung bei 72°C für 10 Sek. Anschließend wurde eine Schmelzkurvenanalyse durchgeführt. Die gemessene Fluoreszenz jedes Durchgangs wurde mit der LightCycler Software ausgewertet. Die relative Menge an Target jeder Probe ergab sich aus dem Vergleich mit der Menge an housekeeping Gen derselben Probe. Die Quantifizierung wurde mit der 2^{-ΔΔC(T)} Methode von Livak und Schmittgen (Livak KJ und Schmittgen TD. Analysis of relative gene expression data using real-time quantitative PCR and the 2(-Delta Delta C(T)) Methods 2001;25:402-8.) durchgeführt.

Die bei den einzelnen Durchgängen der Präparation der Cortischen Organe gewonnenen Mengen an RNS waren im Vergleich zu dem, was beispielsweise aus Regionen des Gehirns üblicherweise gewonnen wird, sehr gering. Deshalb mussten die Targets sorgfältig aufgrund des Standes der wissenschaftlichen Erkenntnisse ausgewählt werden. Daraus ergab sich erstens: Da bei der Verschiedenartigkeit der Erkrankungen des Innenohrs eine einzelne Ursache ausgeschlossen werden kann, sollte ein Medikament ganz allgemein die Degeneration von Nervenzellen, die ja bei allen Erkrankungen des Innenohrs geschädigt sind, aufhalten oder sogar rückgängig machen. Nach dem Stand des Wissens kommen dafür nur Neurotrophine (Wachstumsfaktoren für Nervenzellen) in Frage. Neurotrophine sind natürliche Peptide, die für die Bildung, Differenzierung und die Lebensfähigkeit von Nervenzellen eine maßgebliche Rolle spielen. Dabei sind die einzelnen Nervenzelltypen auf die Präsenz von bestimmten Neurotrophinen besonders angewiesen. Die zweite wissenschaftliche Erkenntnis ist die folgende: Die Schwerhörigkeit beruht vor allem auf einer Störung der Nervenzellen vom Typ der dopaminergen Nerven. Deshalb sollte ein Medikament besonders dopaminerge Mechanismen aktivieren.

Wie in Figur 1 dargestellt, wurde die Bildung mehrerer Neurotrophine aktiviert, von denen bekannt ist, dass sie allgemein neuroprotektive Eigenschaften haben. Dies sind Armetl 1, BDNF, Cbln 1, NGF und NPY (Erklärung der Abkürzungen s. Legende zu Figur 1). Außerdem wurden dopaminerge Proteine vermehrt aktiviert wie beispielsweise der Dopamin Rezeptor Subtyp 1 und die Tyrosinhydroxylase, die Dopamin in den Nervenzellen synthetisiert. Bekannt ist, dass der Dopaminrezeptor 1 derjenige Subtyp ist, der im Innenohr bei weitem am meisten vorkommt. Es wurden mehrere unabhängige Experimente durchgeführt mit nahezu identischen Ergebnissen (Standardabweichung <10%). Die Befunde waren also sehr gut reproduzierbar.

Diese Ergebnisse belegen, dass 9-Me-BC spezifisch charakteristische Proteine von dopaminergen Nervenzellen aktiviert. Bisher ist keine Substanz beschrieben, die ein solches Wirkspektrum hat. Dieses ist insofern besonders günstig, als die Wirkungen im einzelnen gerade den Vorgängen entgegenwirken, die nach heutigem Kenntnisstand bei der Innenohrschwerhörigkeit eine besondere Rolle spielen. Bei 9-Me-BC handelt es sich also um eine Substanz mit einzigartigem Wirkprofil, das besonders geeignet ist, um degenerative Vorgänge bei Nervenzellen, insbesondere vom Typ der dopaminergen Nervenzellen zu stoppen oder sogar rückgängig zu machen.

Die in Figur 2 dargestellten Untersuchungen aus dem Jahr 2005 sind von Th. Lenarz und seinen Mitarbeitern von der Medizinischen Hochschule Hannover erstellt worden, die das weltgrößte Cochlear-Implant Zentrum betreiben. Sie haben das Spiralganglion, also einen Teil des Hörnervs, verschiedenen Substanzen für 48 Stunden ausgesetzt um zu prüfen, welche davon neuroprotektive Eigenschaften haben. Wie in Figur 2 dargestellt, sind dies besonders BDNF und GDNF. Die Schlussfolgerung dieser Studie war, dass Substanzen, die BDNF und GDNF aktivieren, vielversprechende Therapeutika für Schäden des Innenohrs sind. Damals, also 2005, waren solche Substanzen unbekannt. Diese Befunde einer anderen Arbeitsgruppe mit weltweiter Anerkennung unterstreichen demnach das therapeutische Potential von 9-Methyl-BC. Dabei ist anzumerken, dass weder BDNF noch GDNF als große Peptide geeignet sind, selbst lokal appliziert zu werden. 9-Methyl-BC ist dagegen eine relativ kleine Substanz (MG: 183), die lipophil und chemisch stabil ist und, wenn überhaupt, nur langsam metabolisiert wird (s. Bsp. 29).

### Beispiele 15 - 28: Differenzierungsmodell humane Neuroblastomzellen (SH-SY5Y)

Ein wesentlicher Gesichtspunkt für die folgenden Untersuchungen war, dass die bisherigen Beispiele im Tiermodell an Ratten und Meerschweinchen durchgeführt wurden, woraus sich die Aufgabe ergab, zu prüfen, ob die ß-Carboline in humanem Gewebe ebenfalls über die Induktion von Wachstumsfaktoren pro-differenzierend wirken. Deshalb wurden mehrere Experimente an humanen SH-SY5Y Neuroblastomzellen durchgeführt.

Um eine reproduzierbare und abgesicherte Aussage zum Wirkungsspektrum von 9-Me-BC zu erhalten, mussten die Cortischen Organe von mehr als 40 Ratten präpariert und analysiert werden. Dies ist ein enormer Arbeitsaufwand und Bedarf an Tieren. Deshalb wurden die Verbindungen XI, XII, XVIII, XIX sowie C bis L im nachfolgend beschriebenen Differenzierungsmodell untersucht. Da es weder Zelllinien noch Differenzierungsmodelle für Haarzellen des Mittelohrs gibt, mußte auf andere Zelllinien wie beispielsweise Neuroblastomzelllinien zurückgegriffen werden, die ähnliche Differenzierungseigenschaften aufweisen wie die Haarzellen des Mittelohrs.

In diesem Modell werden humane Neuroblastomzellen eingesetzt. Dies sind permanente Zellen, die als Indifferenztyp in Kultur vorliegen. Sie können durch Zugabe von bestimmten Substanzen zum Kulturmedium wie beispielsweise Retinsäure, Brain-derived Nervenwachstumsfaktor (BDNF), oder Aktivatoren des Enzyms Proteinkinase C zur Differenzierung, das heißt zur Umwandlung in neuronale Zellen, angeregt werden. Wir haben dieses Zellsystem angewendet um zu untersuchen, ob die Beta-Carboline die Indifferenzzellen in den neuronalen Typ umwandeln können. Die Kriterien waren: weniger Proliferation (weniger Zellen pro Gesichtsfeld); kleinere Zellkörper (Übergang vom Indifferenztyp zum neuronalen Typ); Zellfortsätze (Neuriten), die Verbindungen zu Nachbarzellen bzw. Cluster von Nachbarzellen herstellen. Daraus ergaben sich die folgenden Ergebnisse.

**Tabelle 1: weitere ß-Carboline im Vergleich zu 9-Methyl-ß-Carbolin**

| 9-Methyl-ß-Carbolin (VI) | **Standard** | |
|---|---|---|
| Fluorethyl-BC (XIX) | + | |
| 9-Me-6-MeO-BC (XVIII) | + | |
| Verbindung C | ± | +: geringfügig besser als 9-Methyl-ß-Carbolin |
| Verbindung D | + | |
| Verbindung (XI) | + | |
| Verbindung (XII) | + | |
| Verbindung E | + | ±: gleich wie 9-Methyl-β-Carbolin |
| Verbindung F | ± | |
| Verbindung G | ± | |
| Verbindung H | + | -: geringfügig schlechter als 9-Methyl-β-Carbolin |
| Harman | - | |
| Verbindung J | ± | |
| Verbindung K | + | |
| Verbindung L | + | |
| Verbindung (XVI) | - | |
| Verbindung (XVII) | ± | |
| Verbindung (VII) | - | |
| Verbindung B | - | |
| Verbindunq (XIII) | ± | |
| Verbindung (XIV) | - | |
| Verbindung A | - | |
| Verbindung (XX) | - | |
| Verbindung (VIII) | ± | |
| Verbindung I | - | |
| Verbindung (XV) | ± | |
| Verbindung (IX) | - | |
| Verbindung (X) | ± | |

Wie aus Tabelle 1 hervorgeht sind die von uns untersuchten Beta-Carboline tatsächlich in der Lage, in unterschiedlichem Ausmaß die Differenzierung zu fördern.

Die Figuren 3 bis 5 ergänzen und veranschaulichen die in Tabelle 1 gezeigten Daten.

Messung der Neuritenlänge als Maß für eine pro-differenzierende Wirkung: Humane SH-SY5Y Neuroblastomzellen wurden für 14 Tage mit Lösungsmittel (Ko) oder Testsubstanz in Kultur inkubiert. Eine Rechtsverschiebung einer Kurve für eine Testsubstanz deutet auf eine Zunahme der Neuritenlänge hin im Vergleich zur Kurve für eine Kontrollsubstanz. Statistische Unterschiede zwischen den Behandlungsgruppen wurden mit *Tukey's Multiple Comparison-Test* berechnet.

Figur 3A zeigt die Neuritenlänge von humanen SH-SY5Y Zellen, die wie oben beschrieben mit Harman behandelt wurde. Der Unterschied zwischen der Kontrollgruppe und der Gruppe, die mit 110 µM Harman behandelt wurden, war signifjkant (P<0,001). Harman führt also zu einer Zunahme der Neuritenlänge.

Figur 3B zeigt die Neuritenlänge von humanen SH-SY5Y Zellen, die wie oben beschrieben mit 9-Methyl-9*H*-ß-Carbolin behandelt wurden. Der Unterschied zwischen der Kontrollgruppe und den Gruppen, die mit 70, 90 oder 110 µM 9-Methyl-9*H*-ß-Carbolin behandelt wurden, war signifjkant (P<0,001). 9-Methyl-9*H*-ß-Carbolin führt also zu einer Zunahme der Neuritenlänge.

Figur 3C zeigt die Neuritenlänge von humanen SH-SY5Y Zellen, die wie oben beschrieben mit 6-Methoxy-9-methyl-9*H*-ß-Carbolin behandelt wurden. Der Unterschied zwischen der Kontrollgruppe und der Gruppe, die mit 50 µM 6-Methoxy-9-methyl-9*H*-ß-Carbolin behandelt wurden, war signifjkant (P<0,001). 6-Methoxy-9-methyl-9*H*-ß-Carbolin führt also zu einer Zunahme der Neuritenlänge.

Figur 3D zeigt die Neuritenlänge von humanen SH-SY5Y Zellen, die wie oben beschrieben mit 9-(2-Fluorethyl)-9*H*-ß-Carbolin behandelt wurden. Der Unterschied zwischen der Kontrollgruppe und den Gruppen, die mit 30, 50 oder 70 µM 9-(2-Fluorethyl)-9*H*-ß-Carbolin behandelt wurden, war signifjkant (P<0,001). 9-(2-Fluorethyl)-9*H*-ß-Carbolin führt also zu einer Zunahme der Neuritenlänge.

Inkubation von humanen SH-SY5Y Neuroblastomzellen für 14 Tage in Kultur führte zu einer Zunahme der Neuritenlänge. Dargestellt sind die kumulativen Häufigkeitsverteilungen der Neuritenlängen, bestimmt in mindestens 20 randomisiert ausgewählten Gesichtsfeldern (jedes 640.000 µm²) von drei unabhängigen Experimenten. Ein steiler Anstieg weist auf, überwiegend, kurze Neurite hin, wie beispielsweise in Kontrollzellen, während ein flacherer Anstieg eine vergleichsweise grössere Anzahl an längeren Neuriten bedeutet. Das horizontale Maximum induziert die maximale Neuritenlänge der jeweiligen Behandlungsgruppe. Für die Kontrollbedingungen sind dies ∼ 500 Mikrometer. Die Ergebnisse zeigen, dass die erfindungsgemäßen β-Carboline das Aussprossen von Neuriten fördern. Die Wirkungen sind dosis- und substanzabhängig: 1-Methyl-ß-Carbolin < 6-Methoxy-9-Methyl-ß-Carbolin < 9-Methyl-ß-Carbolin <9 -Fluorethyl-ß-Carbolin). Die ß-Carboline haben also eine pro-differenzierende Wirkung.

Die Effekte der weiteren erfindungsgemäßen ß-Carboline (Verbindungen A - L und VI - XX) auf die Neuritenlänge wurden wie oben beschrieben in humanen SH-SY5Y Neuroblastomzellen nach 14 Tagen bestimmt. Tabelle 1 faßt die Ergebnisse zusammen.

### Induktion der Bildung von Wachstumsfaktoren:

Als nächstes wurde untersucht, ob die Wirkstoffe in humanen SH-SY5Y Neuroblastomzellen die Bildung von Nerven-Wachstumsfaktoren induzieren. Nach zwei und 14 Tagen wurden die Zellen geerntet und die Expression der Ziel- und Haushaltsgene mit reverser Transkriptions-PCR bestimmt. Figur 5 zeigt Mittelwerte von drei unabhängigen Experimenten zu Nerven-Wachstumsfaktoren, von denen eine regenerierende Wirkung auf geschädigte Hörsinneszellen bekannt ist.

Figur 4A zeigt die relative Genexpression von BDNF (*brain-dereived neurotrophic factor*). Die Transkription von BDNF war nach zwei Tagen nur durch die höchste Konzentration (110 µM) von LE-02 (9-Methyl-ß-Carbolin) stimuliert, während nach 14tägiger Exposition die niedrigen Konzentrationen von 30 und 50 µM eine Stimulierung induzierten. Eine solche Verschiebung wurde auch für 9-Fluorethyl-ß-Carbolin (Substanz Nr. 559; nach 2 Tagen: Stimulierung durch 70 und 90 µM; nach 14 Tage durch 30 und 50 µM). 6-Methoxy-9-Methyl-ß-Carbolin (Substanz Nr. 513) führte zu einem enormen, dosisabhängigen Anstieg der BDNF Transkription.

Figur 4B zeigt die relative Genexpression von Cerebellin-1 Precursor Protein (CBLN). Precursor Protein war nach zweitägiger Exposition nur durch 9-Fluorethyl-ß-Carbolin erhöht. Nach vierzehntägiger Exposition erhöhten alle getesteten β-Carboline die CBLN Expression. Niedrige Konzentrationen der Testsubstanzen wirkten stärker als hohe Konzentrationen mit Ausnahme von 9-Fluorethyl-ß-carbolin, von dem alle untersuchten Konzentrationen etwa gleich stark stimulierten. Cerebellin-1 9-Fluorethyl-ß-carbolin zeigt ähnliche Effekte auf die CBLN Expression wie auf die Neuritenlänge (siehe Figure 3D).

Figur 4C zeigt die relative Genexpression von GDNF (*glial cell line-derived neurotrophic factor*). Die vierzehntägige Exposition führte für einzelne Konzentrationen zu einer bis zu dreifachen Stimulierung.

NGF (nerve growth factor) und NPY (Neuropeptid Y) sind Wachstumsfaktoren, die ganz generell für das Überleben von Nervenzellen erforderlich sind. Die Ausschaltung der Biosynthese führt zum Absterben der Nervenzellen. Sie ermöglichen die Differenzierung, z. B. Synapsenbildung, was für die Anpassung an neue Situationen wie neue Hintergrundgeräusche, neue Melodien usw. unabdingbar ist. Diese Wachstumsfaktoren ermöglichen und steuern auch das erneute Auswachsen von geschädigten Nervenzellfortsätzen und ermöglichen so die Reintegration der neu ausgewachsenen Zellfortsätze in die vorhandenen Nervenbahnen und Schaltkreise. Bei einer akuten und chronischen Hörschädigung sind sie ein wichtiger Bestandteil des natürlichen Reparaturmechanismus.

Figur 4D zeigt die relative Genexpression von NGF (*nerve growth factor*). Nach vierzehntägiger Exposition war die Genexpression 20 bis 50fach erhöht. Auch für NGF gilt, dass die niedrigen Konzentrationen wesentlich wirksamer waren.

Figur 4E zeigt die relative Genexpression von NPY (Neuropeptid Y). Nach vierzehntägiger Exposition war die Genexpression zwischen 1,3 und 4-fach verstärkt. Für 9-Methyl-ß-Carbolin und 9-Fluorethyl-ß-Carbolin waren die niedrigen Konzentrationen wirksamer als die höheren.

Die Effekte der weiteren erfindungsgemäßen ß-Carboline (Verbindungen A - L und VI - XX) auf die Genexpression von BDNF, GDNF, NGF und NPY wurden wie oben beschrieben in humanen SH-SY5Y Neuroblastomzellen nach 2 und 14 Tagen bestimmt. Tabelle 2 faßt die Ergebnisse zusammen.

**Tabelle 2: weitere ß-Carboline im Vergleich zu 9-Methyl-ß-Carbolin**

| 9-Methyl-β-Carbolin | **Standard** | |
|---|---|---|
| Verbindung C | ± | |
| Verbindung D | + | |
| Verbindung (XI) | ± | |
| Verbindung (XII) | + | |
| Verbindung E | ± | |
| Verbindung F | ± | |
| Verbindung G | ± | |
| Verbindunq H | ± | |
| Harman | - | +: geringfügig besser als 9-Methyl-β-Carbolin |
| Verbindung J | - | |
| Verbindung K | - | |
| Verbindung L | ± | |
| Verbindung (XVI) | - | ±: gleich wie 9-Methyl-β-Carbolin |
| Verbindunq (XVII) | ± | |
| Verbindung (VII) | ± | |
| Verbindung B | - | -: geringfügig schlechter als 9-Methyl-β-Carbolin |
| Verbindunq (XIII) | ± | |
| Verbindung (XIV) | - | |
| Verbindung A | - | |
| Verbindung (XX) | ± | |
| Verbindung (VIII) | - | |
| Verbindung I | - | |
| Verbindung (XV) | + | |
| Verbindung (IX) | - | |
| Verbindung (X) | ± | |

Untersuchung der Aktivierung von intrazellulären Kaskaden zur Aufklärung der Transkriptionsfaktoren, die über Getranskription die Differenzierung massgeblich stimulieren:

Die Wirkung von ß-Carbolinen auf mehrere intrazelluläre Kaskaden wurde untersucht. Von diesen ist bekannt, dass an deren Ende bestimmte Transkriptionsfaktoren im Zellkern gebildet werden, die ihrerseits die Transkription einer Gruppe weiterer Gene aktivieren. Dazu wurde die Dual Luciferase Methode verwandt. Eine Luciferase dient als Transfektionskontrolle, während die zweite an die entsprechende spezifische Bindungssequenz der DNA gekoppelt ist.

Figur 5 zeigt, dass die getesteten ß-Carboline (6-Methoxy-9-Methyl-ß-Carbolin, 9-Methyl-ß-Carbolin und 9-Fluorethyl-ß-Carbolin) dosisabhängig die Bildung des Transkriptionsfaktors CREB bis zum 10fachen der Kontrollzellen in SH-SY5Y Zellen aktivieren (drei unabhängige Experimente, 48 h Inkubation).

Die Befunde ergeben eindeutig, dass die untersuchten erfindungsgemäßen β-Carboline pro-differenzierend wirken. Die Induktion der Bildung mehrerer Wachstumsfaktoren, von denen bekannt ist, dass sie neuroprotektiv und in mehreren Schädigungsmodellen auch neuroregenerativ wirken, belegt eine therapeutische Wirkung der Wirkstoffe. Die Wirkung erfolgt durch Aktivierung von intrazellulären Kaskaden, die Transkriptionsfaktoren aktivieren. Diese transkribieren eine Gruppe von Genen, die u. a. für Wachstumsfaktoren kodieren.

### Beispiel 29

### Pharmakokinetische Experimente an einem Tiermodell

In diesem Beispiel wird 9-Methyl-β-Carbolin lokal in die Nische vor der Rundfenstermenbran platziert. Von der Nische aus kann das β-Carbolin durch das Runde Fenster in die Perilymphe (Flüssigkeit, die die Sinneszellen schützt) des Innenohrs (Hörschnecke oder Cochlea) und die Bogengänge des Gleichgewichtsorgans diffundieren.

Eine lokale Applikation vermeidet unerwünschte Wirkungen, die bei systemischer Anwendung zu erwarten sind, weil zum Einen eine vergleichsweise hohe Dosis notwendig ist und zum Anderen die chronische Verabreichung, wie sie bei der Schwerhörigkeit und u. U. bei Schwindel erforderlich ist, das Risiko von unerwünschten Wirkungen erfahrungsgemäß erhöht. Deshalb ist die Voraussetzung für die Anwendung der Substanz, dass sie tatsächlich durch die Membran des Runden Fensters diffundiert.

Um dies zu prüfen wurde 9-Methyl-β-Carbolin ∗ HCl (9-me-BC) in steriler Phosphatgepufferter physiologischer Kochsalzlösung (PBS) gelöst und dann mit einer Flussrate von 0,5 µL/h bis zu 10 Stunden vor die Rundfenstermembran von Meerschweinchen mittels einer osmotischen Minipumpe kontinuierlich infundiert. Die Infusion erfolgte also in das Mittelohr und damit außerhalb der Schnecke des Innenohrs, wobei das Trommelfell nicht beschädigt wurde. Nach drei Stunden wurde erstmals aus dem Apex, der Spitze der Schnecke, also dem am weitesten von der Rundfenstermembran entfernten Teil der Schnecke, 5µL Perilymphe mit einer zuvor implantierten Kanüle abgenommen. Die Probe wurde verdünnt und dann ohne eine weitere Aufreinigung direkt in einem Hochdruck-Flüssigkeitschromatographen (HPLC) aufgetrennt und die natürliche Fluoreszenz des β-Carbolins mit einem Fluoreszenzdetektor gemessen. Bei einem zweiten Meerschweinchen wurde das Experiment mit der gleichen Versuchsanordnung wiederholt, aber nach 10-stündiger Infusion die Probe entnommen. Einem dritten Tier wurde Perilymphe entnommen, ohne dass es zuvor mit 9-me-BC behandelt worden war (Leerwert-Kontrolle).

In einer zweiten Serie wurden 20 µg 9-me-BC durch die Rundfenstermembran hindurch in die Perilymphe direkt appliziert, also intracochleär. Nach 3 Stunden und bei einem weiteren Tier nach 20 Stunden wurde Perilymphe entnommen und gemessen wie oben beschrieben. In Figur 6 sind die Ergebnisse dargestellt zusammen mit einer Eichreihe. Die Eichreihe zeigt, dass 10 pg 9-me-BC mit dieser Methode sicher nachgewiesen werden können. In den Chromatogrammen der oberen Reihe in Figur 6 ist ein einzelnes Maximum bei etwa 7 Minuten zu sehen, das das 9-me-BC darstellt. Es ist eindeutig kein weiteres Maximum zu sehen, das 9-me-BC wird also nicht abgebaut. Außerdem zeigen die Chromatogramme eindeutig, dass 9-me-BC durch die Rundfenstermembran penetriert.

Die beiden Chromatogramme in der mittleren Reihe zeigen, dass nach direkter Applikation in die Perilymphe das 9-me-BC auch noch nach 20 Stunden vorhanden ist. Das ist für die therapeutische Anwendung von eminenter Bedeutung. Außerdem zeigen diese Chromatogramme, dass 9-me-BC auch innerhalb dieser langen Periode weder abgebaut wird noch zerfällt. 9-me-BC ist also chemisch wie metabolisch inert (notabene, in der oberen Reihe werden ng nachgewiesen und in der mittleren Reihe pg; deshalb sind in der mittleren und in der unteren Reihe, linkes Chromatogramm, die sog. Injektionspeaks zu sehen und ein Rauschen der Basislinie).

In der unteren Reihe links ist ein Chromatogramm abgebildet, das zeigt, dass bei einem unbehandelten Tier kein 9-me-BC in der Perilymphe vorhanden ist (Leerwert-Kontrolle). Bei einem unbehandelten Tier handelt es sich um ein Tier, bei dem nur die operativen Eingriffe wie bei den anderen Meerschweinchen vorgenommen wurden, aber kein 9-me-BC appliziert wurde. Die Befunde sind im Vergleich zu anderen Substanzen (Dexamethason, Gentamycin, 2-Methylthiazolidin-2,4-dicarbonsäure [Tialin]) sehr günstig im Sinne der Therapie.

Da sich die Sinneszellen des Hörorgans, nämlich die Haarzellen, sehr ähnlich wie die Sinneszellen des Gleichgewichtsorgans verhalten, sind Medikamente, die zur Behandlung bei Erkrankungen des einen Systems eingesetzt werden, auch bei Erkrankungen des anderen Systems wirksam.

### Beispiel 30

### Prophylaxe von Hörschäden am Tiermodell

Eine β-Carbolin enthaltende Lösung wurde drei Tage lang in die Nische vor der Rundfenstermembran von Meerschweinchen mittels einer osmotischen Pumpe kontinuierlich infundiert. Die folgenden β-Carboline wurden in diesem Experiment individuell getestet: 9-Methyl-β-Carbolin, Verbindungen XI, XII, XVIII, XIX sowie C bis L.

Dann wurde den Meerschweinchen eine Standarddosis von Gentamycin und Etacrynsäure intravenös gespritzt. Gentamycin und Etacrynsäure schädigen das Innenohr. Sie werden als Toxine für solche Untersuchungen üblicherweise verwendet, um Schwerhörigkeit zu erzeugen. Die Infusion wurde noch zwei Tage fortgesetzt. Dann wurde die elektrophysiologische Aktivität der Nervenzellen im Hirnstamm, die die Impulse aus dem Innenohr weiterleiten, nach Beschallung des Ohrs mit 2, 4, 8 und 20 Hz abgeleitet. Das nicht mit β-Carbolin behandelte Ohr diente als intraindividuelle Kontrolle (Prieskorn DM, Miller JM. Technical report: chronic and acute intracochlear infusion in rodents. Hear Res. 2000;140(1-2):212-5). Ausserdem wurden Tiere nur mit 9-me-BC infundiert, aber nicht mit Gentamycin behandelt, als weitere Kontrolle.

Die gemessene elektrophysiologische Aktivität der Nervenzellen des Hörnervs (Hörschwelle) dient als Maß für die therapeutische Wirkung der eingesetzten β-Carboline auf das Innenohr. Wie Tabelle 3 zu entnehmen ist, zeigen die eingesetzten β-Carboline die Steigerung der elektrophysiologischen Aktivität der Nervenzellen des Hörnervs. Daraus kann gefolgert werden, dass ß-Carboline zur Behandlung von Hörschäden, Schwindel und Gleichgewichtsstörungen verwendbar sind.

**Tabelle 3: Wirkung verschiedener β-Carboline auf das Innenohr im Vergleich zu 9-methyl-β-Carbolin**

| 9-Methyl-β-Carbolin | **Standard** | |
|---|---|---|
| Fluorethyl-BC (XIX) | + | |
| 9-Me-6-MeO-BC (XVIII) | ± | |
| Verbindung C | + | |
| Verbindung D | - | |
| Verbindunq (XI) | ± | |
| Verbindung (XII) | + | |
| Verbindung E | - | |
| Verbindung F | ± | |
| Verbindung G | + | |
| Verbindunq H | + | +: geringfügig besser als 9-Methyl-β-Carbolin |
| Harman I | - | |
| Verbindung J | ± | |
| Verbindunq K | ± | |
| Verbindung L | + | ±: gleich wie 9-Methyl-β-Carbolin |
| Verbindung (XVI) | - | |
| Verbindung (XVII) | - | |
| Verbindung (VII) | - | -: geringfügig schlechter als 9-Methyl-β-Carbolin |
| Verbindung B | - | |
| Verbindung (XIII) | ± | |
| Verbindung (XIV) | - | |
| Verbindung A | - | |
| Verbindung (XX) | ± | |
| Verbindung (VIII) | ± | |
| Verbindung I | - | |
| Verbindung (XV) | - | |
| Verbindung (IX) | - | |
| Verbindung (X) | ± | |

### Beispiel 31

### Formulierung von 9-Methyl-β-Carbolin für die topische Applikation

Ein Gelformulierung mit 50 µM 9-Methyl-β-Carbolin für die topische Applikation besteht aus 0,7 Gewichtprozent Hyaluronsäure in phosphatgepufferter Kochsalzlösung, z. B. *Hylumed Sterile* von Genzyme Corp, mit einem Gehalt an 9-Methyl-β-Carbolin von 50 µM.

### Beispiel 32

### Formulierung von 9-Methyl-β-Carbolin für die topische Applikation

Für die topische Applikation von 9-Methyl-β-Carbolin in Form von Ohrentropfen wird eine Lösung von 3 mg/ml 9-Methyl-β-Carbolin in gereinigtem Wasser unter Verwendung der folgenden Hilfsstoffe hergestellt: Benzalkoniumchlorid, Natriumacetat ×3 H₂O, Essigsäure, Mannitol (Ph. Eur.), Natriumedetat (Ph. Eur.) und Salzsäure/Natriumhydroxid (zur pH-Einstellung).

### Beispiel 33

### Formulierung von 9-Methyl-β-Carbolin für die topische Applikation

Zu einer Lösung von 0,1 mg Butylhydroxyanisol (Ph.Eur.) in 939,9 mg Gylcerol werden 100 mg 9-Methyl-β-Carbolin gegeben. Die entstandene Lösung kann topisch im Ohr appliziert werden. Optional können Penetrationsverstärker wie Dimethylsulfoxid zugegeben werden.

### Beispiel 34

### Formulierung von 9-Methyl-β-Carbolin für die orale Applikation

Das Beispiel gibt die Zusammensetzung für eine filmbeschichtete Tablette mit 12,5 mg 9-Methyl-β-Carbolin wieder. Der Tablettenkern setzt sich wie folgt zusammen:

| | |
|---|---|
| 9-Methyl-β-Carbolin | 12,50 mg |
| Cellulose, mikrokristallin | 103,25 mg |
| Croscarmellose-Na | 6 24 mg |
| Siliciumdioxid, kolloidal | 1,25 mg |
| Talcum | 1,25 mg |
| Magnesiumstearat | 0,50 mg |
| **Gesamtgewicht** | **125,0 mg** |

Der Tablettenkern wird mit 5 mg Hydroxypropylmethylcellulose (HPMC) wie beispielsweise *Opadry* oder *Sepifilm* beschichtet. Die resultierende Filmtabelette besitzt ein Gesamtgewicht von 130 mg und enthält neben den genannten Hilfsstoffen 12,5 mg 9-Methyl-β-Carbolin.

### Beispiel 35

### Formulierung von 9-Methyl-β-Carbolin für die orale Applikation

Das Beispiel gibt die Zusammensetzung für eine filmbeschichtete Tablette mit 50,0 mg 9-Methyl-β-Carbolin wieder. Der Tablettenkern setzt sich wie folgt zusammen:

| | |
|---|---|
| 9-Methyl-β-Carbolin | 50,0 mg |
| Cellulose, mikrokristallin | 413,0 mg |
| Croscarmellose-Na | 25,0 mg |
| Siliciumdioxid, kolloidal | 5,0 mg |
| Talcum | 5,0 mg |
| Magnesiumstearat | 2,0 mg |
| **Gesamtgewicht** | **500,0 mg** |

Der Tablettenkern wird mit 20 mg HPMC wie beispielsweise *Opadry* oder *Sepifilm* beschichtet. Die resultierende Filmtabelette besitzt ein Gesamtgewicht von 520 mg und enthält neben den genannten Hilfsstoffen 50,0 mg 9-Methyl-β-Carbolin.

### Beispiel 36

### Formulierung von 6-Methoxy-9-methyl-9H-ß-Carbolin für die orale Applikation

Das Beispiel gibt die Zusammensetzung für eine filmbeschichtete Tablette mit 50,0 mg 6-Methoxy-9-methyl-9*H*-ß-Carbolin wieder. Der Tablettenkern setzt sich wie folgt zusammen:

| | |
|---|---|
| 6-Methoxy-9-methyl-9*H*-ß-Carbolin | 50,0 mg |
| Cellulose, mikrokristallin | 413,0 mg |
| Croscarmellose-Na | 25,0 mg |
| Siliciumdioxid, kolloidal | 5,0 mg |
| Talcum | 5,0 mg |
| Magnesiumstearat | 2,0 mg |
| **Gesamtgewicht** | **500,0 mg** |

Der Tablettenkern wird mit 20 mg HPMC wie beispielsweise *Opadry* oder *Sepifilm* beschichtet. Die resultierende Filmtabelette besitzt ein Gesamtgewicht von 520 mg und enthält neben den genannten Hilfsstoffen 50,0 mg 6-Methoxy-9-methyl-9*H*-ß-Carbolin.

### Beispiel 37

### Formulierung von 9-Fluorethyl-ß-Carbolin für die orale Applikation

Das Beispiel gibt die Zusammensetzung für eine filmbeschichtete Tablette mit 37,5 mg 9-Fluorethyl-ß-Carbolin wieder. Der Tablettenkern setzt sich wie folgt zusammen:

| | |
|---|---|
| 9-Fluorethyl-ß-Carbolin | 37,50 mg |
| Cellulose, mikrokristallin | 309,75 mg |
| Croscarmellose-Na | 18,75 mg |
| Siliciumdioxid, kolloidal | 3,75 mg |
| Talcum | 3,75 mg |
| Magnesiumstearat | 1,5 mg |
| **Gesamtgewicht** | **375,00 mg** |

Der Tablettenkern wird mit 15 mg HPMC wie beispielsweise *Opadry* oder *Sepifilm* beschichtet. Die resultierende Filmtabelette besitzt ein Gesamtgewicht von 390 mg und enthält neben den genannten Hilfsstoffen 37,5 mg 9-Fluorethyl-ß-Carbolin.

### Beispiel 37

### Formulierung von 9-Fluorethyl-ß-Carbolin für die topische Applikation

Ein Milliliter einer Suspension von 250 mg 9-Fluorethyl-ß-Carbolin für die topische Applikation wird aus 250 mg 9-Fluorethyl-ß-Carbolin, 1 mg p-Hydroxybenzoesäuremethylester als Konservierungsmittel, Cetylstearylalkohol, Sorbitanmonolaurat, Polysorbat und gereinigtem Wasser hergestellt. Die entstandenede Suspension kann für die topische Verabreichung verwendet werden.

## Patentansprüche

1. Verbindungen der allgemeinen Formel (I) 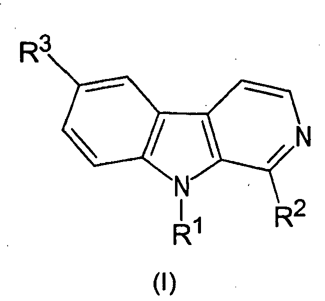 worin
**R¹** für -R¹² steht;
**R²** und **R³** unabhängig voneinander folgende Reste bedeuten: **-R⁷, -R⁸,** -H, -OH, -O**R⁷**, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -F, -Cl, -Br, -I, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -OCF₃, -OC₂F₅,
**R⁷, R⁸** und **R¹²** unabhängig voneinander folgende Reste bedeuten: -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇. -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, ₋C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃. -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -CH=CH-C₂H₄-CH=CH₂, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH=CH-C(CH₃)=CH-CH₃, -CH=C(CH₃)-CH=CH-CH₃, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≅CH, -C=C-CH₃, -CH₂-C≡CH, -C₂H₄-C≡CH, -CH₂-C≡C-CH₃. -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH2-C≡C-C₃H₇, -C=C≡C₄H₉, -C=C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂C≡C-CH₃, -CH(CH₃)-C≡C-C₂H₅, -C≡C-CH(CH₃)-C₂H₅. -C=C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C₁₄H₂₉, -CH₂-CH₂-N(CH₃)₂;
sowie pharmakologisch verträgliche Salze, Solvate, Hydrate, Enantiomere, Diastereomere als auch Racemate der vorgenannten Verbindungen zur Verwendung in der Prophylaxe und Behandlung von Hörschäden, Schwindel und Gleichgewichtsstörungen.

2. Verbindung zur Verwendung gemäß Anspruch 1 der allgemeinen Formel (V) 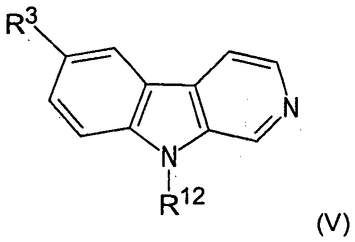 wobei die Reste **R¹²** und **R³** die in Anspruch 1 beschriebene Bedeutung haben.

3. Verbindung zur Verwendung gemäß Anspruch 1 ausgewählt aus der Gruppe bestehend aus: 9-Methyl-9*H*-β-Carbolin, 6-Methoxy-9-iso-Propyl-9*H*-β-Carbolin, 9-[(1*Z*)-1-Methylprop-1-enyl]-9*H*-β-Carbolin, 1-Chlor-9-[(1*Z*,3*E*)-2-Methylpenta-1,3-dienyl]-9*H*-β-Carbolin, 9-Methyl-6-Propoxy-9*H*-β-Carbolin, 9-Cyclopropyl-6-Methoxy-9*H*-β-Carbolin, 6-Trifluormethoxy-9-Methyl-9*H*-β-Carbolin, 6-Dimethylamino-9-Methyl-9*H*-β-Carbolin, 1-Methoxy-6-Chlor-9-Methyl-9*H*-β-Carbolin, 1,9-Dimethyl-9*H*-β-Carbolin, 1-Isopropyl-6,9-Dimethyl-9*H*-β-Carbolin, 6,9-Dimethyl-9*H*-β-Carbolin, 6-Methoxy-9-Methyl-9*H*-β-Carbolin, 9-(2-Fluorethyl)-9*H*-β-Carbolin und 9-Allyl-9*H*-β-Carbolin.

4. Verbindung zur Verwendung gemäß eines der Ansprüche 1 - 3, wobei es sich bei den akuten und chronischen Ohrenerkrankungen und Hörschäden, Schwindel und Gleichgewichtsstörungen insbesondere von Hörsturz, Knalltrauma, Explosiontrauma, Innenohrschwerhörigkeit durch chronische Lärmexposition, Altersschwerhörigkeit, Trauma während der Implantation von Innenohrprothesen (Insertionstrauma), Schwindel aufgrund von Erkrankungen im Innenohrbereich, Schwindel im Zusammenhang und/oder als Symptom der Menière'schen Erkrankung, Gleichgewichtsstörungen im Zusammenhang und/oder als Symptom der Menière'schen Erkrankung, Tinnitus und Hörschäden aufgrund von Antibiotika und
Zytostatika handelt.

5. Pharmazeutische Zusmmensetzung enthaltend mindestens eine Verbindung der Formel (I) zur Verwendung gemäß eines der Ansprüche 1 - 3 in Form von Tropfen, Salben, Sprays, Liposomen,
Gelen, Emulsionen oder Injektionslösungen für die Behandlung von Ohrenerkrankungen und Hörschäden.

## Claims

1. Compounds of the general formula (I) 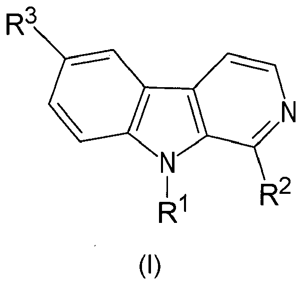 wherein
**R¹** stands for -R¹²;
**R²** and **R³** represent independently of each other the following residues: **-R⁷, -R⁸,** -H, -OH, -O**R⁷**, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -F, -Cl, -Br, -I, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -OCF₃, -OC₂F₅,
**R⁷, R⁸** and **R¹²** represent independently of each other the following residues: -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, Cyclo-C₄H₇, cyclo-C₅H₉, cyclo-C₆H₁₁, cyclo-C₇H₁₃, cyclo-C₈H₁₅. -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH₂, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, ₋C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, ₋CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -CH=CH-C₂H₄-CH=CH₂, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH=CH-C(CH₃)=CH-CH₃, -CH=C(CH₃)-CH=CH-CH₃, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C=CH, -C=C-CH₃, -CH₂-C=CH, -C₂H₄-C=CH, -CH₂-C=C-CH₃, -C=C-C₂H₅, -C₃H₆-C=CH, -C₂H₄-C=C-CH₃, -CH₂-C=C-C₂H₅, -C=C-C₃H₇, -CH(CH₃)-C=CH, -CH₂-CH(CH₃)-C=CH, -CH(CH₃)-CH₂-C=CH, -CH(CH₃)-C=C-CH₃, -C₄H₈-C=CH, -C₃H₆-C=C-CH₃, -C₂H₄-C=C-C₂H₅, -CH₂-C=C-C₃H₇, -C=C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C₁₄H₂₉, -CH₂-CH₂-N(CH₃)₂;
as well as pharmacologically acceptable salts, solvates, hydrates, enantiomers, diastereomers and racemates of the afore-mentioned compounds for use in the prophylaxis and treatment of hearing damages, vertigo and vestibular disorders.

2. Compound for use according to claim 1 of the general formula (V) 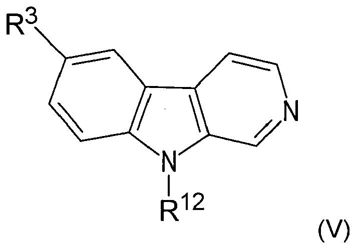 wherein the residues **R¹²** and **R³** have the meaning described in claim 1.

3. Compound for use according to claim 1 selected from the group consisting of: 9-methyl-9*H*-β-carboline, 6-methoxy-9-iso-propyl-9*H*-β-carboline, 9-[(1*Z*)-1-methylprop-1-enyl]-9*H*-β-carboline, 1-chloro-9-[(1*Z*,3*E*)-2-methylpenta-1,3-dienyl]-9*H*-β-carboline, 9-methyl-6-propoxy-9*H*-β-carboline, 9-cyclopropyl-6-methoxy-9*H*-β-carboline, 6-trifluormethoxy-9-methyl-9*H*-β-carboline, 6-dimethylamino-9-methyl-9*H*-β-carboline, 1-methoxy-6-chloro-9-methyl-9*H*-β-carboline, 1,9-dimethyl-9*H*-β-carboline, 1-isopropyl-6,9-dimethyl-9*H*-β-carboline, 6,9-dimethyl-9*H*-β-carboline, 6-methoxy-9-methyl-9*H*-β-carboline, 9-(2-fluorethyl)-9*H*-β-carboline and 9-allyl-9*H*-β-carboline.

4. Compound for use according to one of the claims 1-3, wherein the acute and chronic ear disorders and hearing damages, vertigo and vestibular disorders are in particular hearing loss, acoustic trauma, explosion trauma, labyrinthine deafness due to chronic noise exposure, presbycusia, trauma during implantation of inner ear prosthesis (insertion trauma), vertigo due to diseases of the inner ear, vertigo in relation with and/or as a symptom of Ménière's disease, vestibular disorders in relation with and/or as symptom of Ménière's disease, tinnitus and hearing damages due to antibiotics and cytostatics.

5. Pharmaceutical composition containing at least one compound of general formula (I) for use according to one of the claims 1-3 in form of drops, ointments, sprays, liposomes, gels, emulsions or injection solutions for the treatment of ear diseases and hearing damages.

## Revendications

1. Composés de formule générale (I) 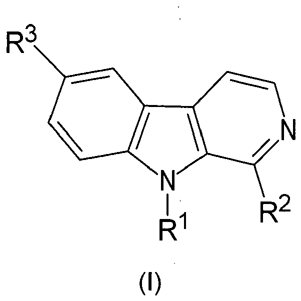 où
**R¹** représente -R¹²;
**R²** et **R³** représentent indépendamment l'un de l'autre les résidus suivants: -**R⁷**, -**R⁸**, -H, -OH, -O**R⁷**, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -F, -Cl, -Br, -I, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -OCF₃, -OC₂F₅,
**R⁷**, **R⁸** et **R¹²** représentent indépendamment l'un de l'autre les résidus suivants: -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH₂, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -CH₂-CH₂-CH₂-OCH₃, -C₂H₄-CH(CH₃)-CH=CH₂, -CH₂-CH(CH₃)-CH₂-CH=CH₂, -CH₂NH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂OH, -CH₂-CH=CH-CH(CH₃)₂, -CH₂-CH=C(CH₃)-C₂H₅, -CH₂-CH₂-CH₂NH₂, -CH₂-C(CH₃)=CH-C₂H₅, -CH(CH₃)-CH=CH-C₂H₅, -CH₂-CH₂NH₂, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH(CH₃)-CH₂-C(CH₃)=CH₂, -CH(CH₃)-CH(CH₃)-CH=CH₂, -CH₂-CH₂-CH₂OH, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -CH=CH-C₂H₄-CH=CH₂, -CH₂-CH=CH-CH=CH-CH₃, -CH=CH-CH₂-CH=CH-CH₃, -CH₂-CH₂-OCH₃, -CH=CH-CH=CH-C₂H₅, -CH₂-CH=CH-C(CH₃)=CH₂, -CH₂-CH₂OH, -CH₂-CH=C(CH₃)-CH=CH₂, -CH₂-C(CH₃)=CH-CH=CH₂, -CH₂-OCH₃, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, ₋C(CH₃)=CH-CH₂-CH=CH₂, -CH=CH-CH=C(CH₃)₂, -CH=CH-C(CH₃)=CH-CH₃, -CH=C(CH₃)-CH=CH-CH₃, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C≡C-C(CH₃)₃, -C₂H₄-CH(CH₃)-C≡CH, -CH₂-CH(CH₃)-CH₂-C≡CH, -CH₂-C≡C-CH(CH₃)₂, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C₁₄H₂₉, -CH₂-CH₂-N(CH₃)₂;
ainsi que des sels pharmacologiquement acceptables, des solvates, des hydrates, des énantiomères, des diastéréoisomères et des racémates des composés mentionnés précédemment pour l'utilisation dans la prophylaxie et le traitement des dommages auditives, du vertige et des troubles vestibulaires.

2. Composé à l'utilisation selon la revendication 1 de formule générale (V) 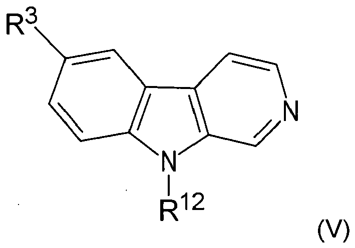 où les résidus R¹² et R³ ont la signification définie dans la revendication 1.

3. Composé à l'utilisation selon la revendication 1 choisi dans le groupe consistant en: 9-méthyl-9*H*-β-carboline, 6-méthoxy-9-iso-propyl-9*H*-β-carboline, 9-[(1*Z*)-1-méthylprop-1-ényl]-9*H*-β-carboline, 1 -chloro-9-[(1*Z*,3*E*)-2-méthylpenta-1,3-diényl]-9*H*-β-carboline, 9-méthyl-6-propoxy-9*H*-β-carboline, 9-cyclopropyl-6-méthoxy-9*H*-β-carboline, 6-trifluorméthoxy-9-méthyl-9*H*-β-carboline, 6-diméthylamino-9-méthyl-9*H*-β-carboline, 1-méthoxy-6-chloro-9-méthyl-9*H*-β-carboline, 1,9-diméthyl-9*H*-β-carboline, 1-isopropyl-6,9-diméthyl-9*H*-β-carboline, 6,9-diméthyl-9*H*-β-carboline, 6-méthoxy-9-méthyl-9*H*-β-carboline, 9-(2-fluorethyl)-9*H*-β-carboline et 9-allyl-9*H*-β-carboline.

4. Composé à l'utilisation selon une des revendications 1-3, où les troubles aiguées et chroniques de l'oreille et les troubles vestibulaires sont en particulier la surdité brusque, le traumatisme sonore, le traumatisme par explosion, la surdité de l'oreille interne à cause de l'exposition au bruit chronique, la presbyacousie, le traumatisme pendant l'implantation d'une prothèse de l'oreille interne (le traumatisme par insertion), le vertige à cause des maladies de l'oreille interne, le vertige en relation avec et/ou comme symptôme de la maladie de Ménière, les troubles vestibulaires en relation avec et/ou comme symptôme de la maladie de Ménière, l'acouphène et les dommages auditives à cause des antibiotiques ou des cytostatiques.

5. Composition pharmaceutique contenant au moins un composé de formule générale (I) à l'utilisation selon une des revendications 1 - 3 sous forme de gouttes, onguents, sprays, liposomes, gels, émulsions ou solutions d'injection pour le traitement des maladies de l'oreille et des dommages auditives.
